# EUROPEAN PATENT APPLICATION

(11) **EP 4 428 239 A1**
(43) Date of publication of application: **11.09.2024**
(21) Application number: 22887213.1
(22) Date of filing: 31.10.2022
(51) Int. Cl.: C12N 15/13, A61K 39/395, A61P 17/04, A61P 25/04, A61P 29/00, A61P 29/02, C07K 16/18, C07K 16/28, C12N 15/63

(54) **NOVEL NAV1.7 MONOCLONAL ANTIBODY**

(30) Priority: 01.11.2021 JP 2021178982; 18.01.2022 JP 2022005967
(71) Applicant: Shionogi & Co., Ltd, Osaka-shi, Osaka 541-0045 (JP)
(72) Inventor: YOSHIKAWA, Mai, Osaka-shi, Osaka 541-0045 (JP); ONODA, Junji, Osaka-shi, Osaka 541-0045 (JP); NAKAMORI, Daiki, Osaka-shi, Osaka 541-0045 (JP); TAKAHASHI, Tatsuya, Osaka-shi, Osaka 541-0045 (JP); KASAI, Erika, Osaka-shi, Osaka 541-0045 (JP)
(74) Representative: Mewburn Ellis LLP
(86) International application number: PCT/JP2022/040580
(87) International publication number: WO 2023/074888

(57) **Abstract**

Objective of the present invention is to provide a novel Nav1.7 monoclonal antibody. It discloses a Nav1.7 monoclonal antibody or its antibody fragment, having specific six CDRs or specific heavy chain variable regions/light chain variable regions. The monoclonal antibody and the like can be used for treating or preventing pain, pruritus and so on.

## Description

### [TECHNICAL FIELD]

The present invention relates to a novel Nav1.7 monoclonal antibody or its antibody fragment. More particularly, the present invention relates to an antibody or its antibody fragment that specifically binds to Navi .7. More particularly, the present invention relates to a monoclonal antibody or its antibody fragment that selectively inhibits Nav1.7 or its antibody fragment, or a pharmaceutical composition containing this one or a kit for detecting Nav1.7.

### [BACKGROUND ART]

Nav1.7 is a voltage-gated sodium ion channel encoded by gene SCN9A, and is expressed primarily in peripheral nerves (Non-Patent Literature: 1). Nav1.7 comprises four domains A, B, C and D (also referred to as domains I-IV), each comprising six transmembrane protein helices (S1, S2, S3, S4, S5 and S6) and three extracellular (hydrophilic) loops E1, E2 and E3 (also referred to as extracellular regions E1, E2 and E3).

It is known that the inflammatory pain is reduced in a Nav1.7 knockout mouse (Non-Patent Literature: 2), and is confirmed that Nav1.7 inhibitor that is a low-molecular compound is effective in erythromelalgia (Non-Patent Literature: 3), trigeminal neuralgia (Non-Patent literature: 4) and the like.

Also, a Nav1.7 monoclonal antibody that is effective for pain (algesic) and pruritus (itch) is reported in Patent Literatures: 1 to 10, etc. Antibodies that bind to the E3 extracellular region of human Nav 1.7 domain C are described in Patent Literatures: 1 to 6 and 8.

### [PRIOR ART REFERENCES]

### [Patent Literatures]

[Patent Literature: 1] WO2011/051350
[Patent Literature: 2] US8734798
[Patent Literature: 3] US8986954
[Patent Literature: 4] US9266953
[Patent Literature: 5] WO2014/159595
[Patent Literature: 6] WO2015/032916
[Patent Literature: 7] WO2015/035173
[Patent Literature: 8] WO2019/230856
[Patent Literature: 9] WO2022/109102
[Patent Literature: 10] WO2011/051349

### [Non-Patent Literatures]

[Non-Patent Literature: 1] PNAS(1997)94:1527-1532
[Non-patent Document: 2] PNAS(2004)101:12706-12711
[Non-patent Document: 3] Pain(2012)153(1):80-85
[Non-patent Document: 4] THE LANCET NEUROLOGY(2017)16(4):291-300

### [SUMMARY OF THE INVENTION]

### [PROBLEMS TO BE SOLVED BY THE INVENTION]

An object of the present invention is to provide a novel Nav1.7 monoclonal antibody or its antibody fragment that can be used as therapeutic agent for pain, pruritus, and the like.

### [MEANS FOR SOLVING THE PROBLEMS]

The present inventors have conducted diligent studies, selected "1424-QPKYEYSL (SEQ ID NO: 1)-1431; hCE3C peptide" as the antigen as described in Example 1 below, and found a monoclonal antibody that specifically binds to the E3 extracellular loop C-terminal region of domain C of Nav1.7 and selectively inhibits Nav1.7. Furthermore, the inventors found that as the below Example 9 describes, the monoclonal antibody of the present invention has a pain inhibitory effect. Also, Patent Literature 7 shows the pruritus inhibitory effects of a Nav1.7 monoclonal antibody, suggesting that the monoclonal antibody of the present invention has a similar pruritus inhibitory effect.

Patent Documents 1 to 4 disclose peptides (C31, C32, and C33) derived from loop E3 of human Nav1.7 domain C, with which rabbits were immunized (TABLE 1). Among them, C33 is a peptide derived from the C-terminus of loop E3 of human Nav1.7 domain C, like the immunogen of the monoclonal antibody of the present inventio or its antibody fragment. However, Patent Documents 1 to 4 do not disclose any antibodies obtained by C33 immunization and the affinity or biological data regarding the antibodies.

Patent Document 5 discloses 38 antibodies obtained using a peptide derived from human Nav1.7 extracellular loop 3-3 (E3 extracellular loop of domain C) as an immunogen. However, the sequence of the peptide has not been disclosed, and it is unknown whether it is the full length or a fragment of the extracellular loop 3-3. Only H4H468P, H4H468B, H4H471P, H4H471B and H1M852N are evaluated for affinity with the extracellular loop 3-3 peptide (residues 1333-1382 of SEQ ID NO: 670 (human Nav1.7 amino acid sequence) of Patent Document 5), specificity to Nav1.7, and the like. Among them, only H4H468P (REGN1064), which is described as a candidate antibody, is evaluated in vivo. However, [0213] and [0214] of Patent Document 5 state that intraperitoneal administration of H4H468P to rats does not affect the mechanical stimulation threshold and the thermal nociceptive threshold 48 hours after administration, and the pain inhibitory effect of H4H468P is not shown.

Patent Document 6 discloses antibodies (10B6 and 10C4) obtained using the hNav1.7-loopC3-llama Fc fusion as an immunogen. Here, hNav1.7-loopC3 is the full-length sequence of the E3 extracellular loop of domain C of hNav1.7, and no antibody against the peptide derived from the C-terminus of hNav1.7-loopC3 is not disclosed. Furthermore, 10B6 and 10C4 are evaluated for affinity and function in vitro but not evaluated at all in vivo.

Patent Document 7 discloses an antibody (SVmab1) binding to domain II (domain B) of Nav1.7 and having the effect of inhibitory pain and pruritus.

Patent Documents 9 and 10 disclose antibodies that bind to domain I (domain A) of Nav1.7.

In other words, Patent Documents 1 to 7, 9, and 10 do not disclose any antibodies that are shown to specifically bind to the C-terminal region of the E3 extracellular loop of human Nav1.7 domain C and to have a pain inhibitory effect.

Patent Document 8, as described in Example 1 shows an antibody whose immunogen is prepared using a peptide of the E3 extracellular loop C-terminus of human Nav1.7 domain C and having an in vivo pain inhibitory effect. Moreover, the immunogen described in Patent Document 8 is prepared by selecting "1418-SVNVDKQPKYEYSL (SEQ ID NO: 1 of Patent Document 8; SEQ ID NO: 103 in the description of the present application)-1431; hCE3C peptide" as an antigen and is not the same as the immunogen of the monoclonal antibodies of the invention.

Specifically, this present invention relates to:
(1) A monoclonal antibody or its antibody fragment that binds to Nav1.7, having;
   a heavy chain variable region including
   a CDR1 having the amino acid sequence of SEQ ID NO: 75: Xaa1-Y-N-M-H (wherein Xaa1 is D or E) optionally having deletion, substitution, insertion and/or addition of 1 to 2 amino acids,
   a CDR2 having the amino acid sequence of SEQ ID NO: 76: R-I-N-P-K-N-G-V-I-Xaa1-Xaa2-N-E-K-F-K-D (wherein Xaa1 is N, L, Q or K and Xaa2 is S, L or Y) optionally having deletion, substitution, insertion and/or addition of 1 to 2 amino acids, and
   a CDR3 having the amino acid sequence of SEQ ID NO: 77: S-Y-Xaa1-G-G-Xaa2-Xaa3-D-A-Y (wherein Xaa1 is Y or F, Xaa2 is N, D, Q or K, Xaa3 is T, D, S or Q) optionally having deletion, substitution, insertion and/or addition of 1 to 2 amino acids, and
   a light chain variable region including
   a CDR1 having the amino acid sequence of SEQ ID NO: 78: R-Xaa1-S-Xaa2-S-V-D-N-Y-G-Xaa3-S-F-Xaa4-N (wherein Xaa1 is A or V, Xaa2 is E or D, Xaa3 is I or F, Xaa4 is M or L) optionally having deletion, substitution, insertion and/or addition of 1 to 2 amino acids,
   a CDR2 having the amino acid sequence of SEQ ID NO: 9 optionally having deletion, substitution, insertion and/or addition of 1 to 2 amino acids, and
   a CDR3 having the amino acid sequence of SEQ ID NO: 10 optionally having deletion, substitution, insertion and/or addition of 1 to 2 amino acids.
(2) The monoclonal antibody or its antibody fragment according to (1),
   having;
   a heavy chain variable region including
   a CDR1 having the amino acid sequence of SEQ ID NO: 4 or 58 optionally having deletion, substitution, insertion and/or addition of 1 to 2 amino acids,
   a CDR2 having the amino acid sequence of any one of SEQ ID NOs: 5, 59 to 63 optionally having deletion, substitution, insertion and/or addition of 1 to 2 amino acids, and
   a CDR3 having the amino acid sequence of any one of SEQ ID NOs: 6, 64 to 71 optionally having deletion, substitution, insertion and/or addition of 1 to 2 amino acids, and
   a light chain variable region including
   a CDR1 having the amino acid sequence of any one of SEQ IDs NO: 8, 72 to 74 optionally having deletion, substitution, insertion and/or addition of 1 to 2 amino acids,
   a CDR2 having the amino acid sequence of SEQ ID NO: 9 optionally having deletion, substitution, insertion and/or addition of 1 to 2 amino acids, and
   a CDR3 having the amino acid sequence of SEQ ID NO: 10 optionally having deletion, substitution, insertion and/or addition of 1 to 2 amino acids.
(3) The monoclonal antibody or its antibody fragment according to (2),
   having
   1) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   2) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   3) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   4) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   5) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   6) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   7) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   8) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   9) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   10) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution,
      insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   11) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   12) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10optionally having deletion, substitution, insertion and/or addition of 1 to 2 amino acids;
   13) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   14) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 64 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   15) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   16) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   17) a heavy chain variable region including
      a CDR1 having the amino acid sequence of SEQ ID NO: 4 optionally having deletion, substitution,
      insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   18) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   19) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 65 optionally having deletion, substitution,
      insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   20) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 66 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   21) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   22) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   23) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 67 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   24) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 60 optionally having deletion, substitution, insertion and/or addition of 1 to 2 amino acids, and
      a CDR3 having the amino acid sequence of SEQ ID NO: 67 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   25) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   26) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   27) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   28) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ NO: 67 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   29) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 59 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   30) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 65 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   31) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 60 optionally having deletion, substitution, insertion and/or addition of 1 to 2 amino acids, and
      a CDR3 having the amino acid sequence of SEQ ID NO: 67 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   32) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 67 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   33) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 60 optionally having deletion, substitution, insertion and/or addition of 1 to 2 amino acids, and
      a CDR3 having the amino acid sequence of SEQ ID NO: 67 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   34) a heavy chain variable region including
      a CDR1 having the amino acid sequence of SEQ ID NO: 58 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 68 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   35) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 69 optionally having deletion, substitution,
      insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   36) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 61 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   37) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 69 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   38) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 62 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   39) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 63 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   40) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 61 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 73 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   41) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 70 optionally having deletion, substitution, insertion and/or addition of 1 to 2 amino acids, and
      a light chain variable region including
      a CDR1 having the amino acid sequence of SEQ ID NO: 72 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   42) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution,
      insertion and/or addition of 1 to 2 amino acids, and
      a CDR3 having the amino acid sequence of SEQ ID NO: 71 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids;
   43) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 74 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids; or
   44) a heavy chain variable region including
      a CDR1 having the amino acid sequence of SEQ ID NO: 4 optionally having deletion, substitution, insertion and/or addition of 1 to 2 amino acids,
      a CDR2 having the amino acid sequence of SEQ ID NO: 5 optionally having deletion, substitution, insertion and/or addition of 1 to 2 amino acids, and
      a CDR3 having the amino acid sequence of SEQ ID NO: 6 optionally having deletion, substitution, insertion and/or addition of 1 to 2 amino acids, and
      a light chain variable region including
      a CDR1 having the amino acid sequence of SEQ ID NO: 8 optionally having deletion, substitution, insertion and/or addition of 1 to 2 amino acids,
      a CDR2 having the amino acid sequence of SEQ ID NO: 9 optionally having deletion, substitution, insertion and/or addition of 1 to 2 amino acids, and
      a CDR3 having the amino acid sequence of SEQ ID NO: 10 optionally having deletion, substitution, insertion and/or addition of 1 to 2 amino acids.
(4) The monoclonal antibody or its antibody fragment according to any one of (1) to (3) wherein the monoclonal antibody is the humanized monoclonal antibody.
(5) The monoclonal antibody or its antibody fragment according to (4),
   having
   a heavy chain variable region having
   the amino acid sequence of any one of SEQ ID NOs: 25, 32 to 54 or
   the amino acid sequence at least 95% identical to the amino acid sequence of any one of SEQ ID NOs: 25, 32 to 54, and
   a light chain variable region having
   the amino acid sequence of any one of SEQ ID NOs: 28, 55 to 57, 79 to 102 or
   the amino acid sequence at least 95% identical to the amino acid sequence of any one of SEQ ID NOs: 28, 55 to 57, 79 to 102.
(6) The monoclonal antibody or its antibody fragment according to (5), having
   1) a heavy chain variable region having the amino acid sequence at least 95% identical to the amino acid sequence of SEQ ID NO: 32, and
      a light chain variable region having the amino acid sequence at least 95% identical to the amino acid sequence of SEQ ID NO: 55;
   2) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 55;
   3) a heavy chain variable region having the amino acid sequence at least 95% identical to the amino acid sequence of SEQ ID NO: 32, and
      a light chain variable region having the amino acid sequence at least 95% identical to the amino acid sequence of SEQ ID NO: 28;
   4) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 28;
   5) a heavy chain variable region having the amino acid sequence at least 95% identical to the amino acid sequence of SEQ ID NO: 34, and
      a light chain variable region having the amino acid sequence at least 95% identical to the amino acid sequence of SEQ ID NO: 55;
   6) a heavy chain variable region having the amino acid sequence at least 95% identical to the amino acid sequence of SEQ ID NO: 35, and
      a light chain variable region having the amino acid sequence at least 95% identical to the amino acid sequence of SEQ ID NO: 55;
   7) a heavy chain variable region having the amino acid sequence at least 95% identical to the amino acid sequence of SEQ ID NO: 36, and
      a light chain variable region having the amino acid sequence at least 95% identical to the amino acid sequence of SEQ ID NO: 55;
   8) a heavy chain variable region having the amino acid sequence at least 95% identical to the amino acid sequence of SEQ ID NO: 37, and
      a light chain variable region having the amino acid sequence at least 95% identical to the amino acid sequence of SEQ ID NO: 28;
   9) a heavy chain variable region having the amino acid sequence at least 95% identical to the amino acid sequence of SEQ ID NO: 37, and
      a light chain variable region having the amino acid sequence at least 95% identical to the amino acid sequence of SEQ ID NO: 55;
   10) a heavy chain variable region having the amino acid sequence at least 95% identical to the amino acid sequence of SEQ ID NO: 36, and
      a light chain variable region having the amino acid sequence at least 95% identical to the amino acid sequence of SEQ ID NO: 28;
   11) a heavy chain variable region having the amino acid sequence at least 95% identical to the amino acid sequence of SEQ ID NO: 38, and
      a light chain variable region having the amino acid sequence at least 95% identical to the amino acid sequence of SEQ ID NO: 55;
   12) a heavy chain variable region having the amino acid sequence at least 95% identical to the amino acid sequence of SEQ ID NO: 34, and
      a light chain variable region having the amino acid sequence at least 95% identical to the amino acid sequence of SEQ ID NO: 28;
   13) a heavy chain variable region having the amino acid sequence at least 95% identical to the amino acid sequence of SEQ ID NO: 39, and
      a light chain variable region having the amino acid sequence at least 95% identical to the amino acid sequence of SEQ ID NO: 55;
   14) a heavy chain variable region having the amino acid sequence at least 95% identical to the amino acid sequence of SEQ ID NO: 35, and
      a light chain variable region having the amino acid sequence at least 95% identical to the amino acid sequence of SEQ ID NO: 28;
   15) a heavy chain variable region having the amino acid sequence at least 95% identical to the amino acid sequence of SEQ ID NO: 38, and
      a light chain variable region having the amino acid sequence at least 95% identical to the amino acid sequence of SEQ ID NO: 28;
   16) a heavy chain variable region having the amino acid sequence at least 95% identical to the amino acid sequence of SEQ ID NO: 39, and
      a light chain variable region having the amino acid sequence at least 95% identical to the amino acid sequence of SEQ ID NO: 28;
   17) a heavy chain variable region having the amino acid sequence at least 95% identical to the amino acid sequence of SEQ ID NO: 40, and
      a light chain variable region having the amino acid sequence at least 95% identical to the amino acid sequence of SEQ ID NO: 55;
   18) a heavy chain variable region having the amino acid sequence at least 95% identical to the amino acid sequence of SEQ ID NO: 40, and
      a light chain variable region having the amino acid sequence at least 95% identical to the amino acid sequence of SEQ ID NO: 56;
   19) a heavy chain variable region having the amino acid sequence at least 95% identical to the amino acid sequence of SEQ ID NO: 41, and
      a light chain variable region having the amino acid sequence at least 95% identical to the amino acid sequence of SEQ ID NO: 55;
   20) a heavy chain variable region having the amino acid sequence at least 95% identical to the amino acid sequence of SEQ ID NO: 40, and
      a light chain variable region having the amino acid sequence at least 95% identical to the amino acid sequence of SEQ ID NO: 28;
   21) a heavy chain variable region having the amino acid sequence at least 95% identical to the amino acid sequence of SEQ ID NO: 42, and
      a light chain variable region having the amino acid sequence at least 95% identical to the amino acid sequence of SEQ ID NO: 55;
   22) a heavy chain variable region having the amino acid sequence at least 95% identical to the amino acid sequence of SEQ ID NO: 41, and
      a light chain variable region having the amino acid sequence at least 95% identical to the amino acid sequence of SEQ ID NO: 28;
   23) a heavy chain variable region having the amino acid sequence at least 95% identical to the amino acid sequence of SEQ ID NO: 43, and
      a light chain variable region having the amino acid sequence at least 95% identical to the amino acid sequence of SEQ ID NO: 55;
   24) a heavy chain variable region having the amino acid sequence at least 95% identical to the amino acid sequence of SEQ ID NO: 44, and
      a light chain variable region having the amino acid sequence at least 95% identical to the amino acid sequence of SEQ ID NO: 55;
   25) a heavy chain variable region having the amino acid sequence at least 95% identical to the amino acid sequence of SEQ ID NO: 42, and
      a light chain variable region having the amino acid sequence at least 95% identical to the amino acid sequence of SEQ ID NO: 28;
   26) a heavy chain variable region having the amino acid sequence at least 95% identical to the amino acid sequence of SEQ ID NO: 45, and
      a light chain variable region having the amino acid sequence at least 95% identical to the amino acid sequence of SEQ ID NO: 56;
   27) a heavy chain variable region having the amino acid sequence at least 95% identical to the amino acid sequence of SEQ ID NO: 46, and
      a light chain variable region having the amino acid sequence at least 95% identical to the amino acid sequence of SEQ ID NO: 56;
   28) a heavy chain variable region having the amino acid sequence at least 95% identical to the amino acid sequence of SEQ ID NO: 43, and
      a light chain variable region having the amino acid sequence at least 95% identical to the amino acid sequence of SEQ ID NO: 28;
   29) a heavy chain variable region having the amino acid sequence at least 95% identical to the amino acid sequence of SEQ ID NO: 46, and
      a light chain variable region having the amino acid sequence at least 95% identical to the amino acid sequence of SEQ ID NO: 28;
   30) a heavy chain variable region having the amino acid sequence at least 95% identical to the amino acid sequence of SEQ ID NO: 45, and
      a light chain variable region having the amino acid sequence at least 95% identical to the amino acid sequence of SEQ ID NO: 55;
   31) a heavy chain variable region having the amino acid sequence at least 95% identical to the amino acid sequence of SEQ ID NO: 44, and
      a light chain variable region having the amino acid sequence at least 95% identical to the amino acid sequence of SEQ ID NO: 28;
   32) a heavy chain variable region having the amino acid sequence at least 95% identical to the amino acid sequence of SEQ ID NO: 43, and
      a light chain variable region having the amino acid sequence at least 95% identical to the amino acid sequence of SEQ ID NO: 56;
   33) a heavy chain variable region having the amino acid sequence at least 95% identical to the amino acid sequence of SEQ ID NO: 47, and
      a light chain variable region having the amino acid sequence at least 95% identical to the amino acid sequence of SEQ ID NO: 55;
   34) a heavy chain variable region having the amino acid sequence at least 95% identical to the amino acid sequence of SEQ ID NO: 48, and
      a light chain variable region having the amino acid sequence at least 95% identical to the amino acid sequence of SEQ ID NO: 55;
   35) a heavy chain variable region having the amino acid sequence at least 95% identical to the amino acid sequence of SEQ ID NO: 49, and
      a light chain variable region having the amino acid sequence at least 95% identical to the amino acid sequence of SEQ ID NO: 55;
   36) a heavy chain variable region having the amino acid sequence at least 95% identical to the amino acid sequence of SEQ ID NO: 50, and
      a light chain variable region having the amino acid sequence at least 95% identical to the amino acid sequence of SEQ ID NO: 55;
   37) a heavy chain variable region having the amino acid sequence at least 95% identical to the amino acid sequence of SEQ ID NO: 49, and
      a light chain variable region having the amino acid sequence at least 95% identical to the amino acid sequence of SEQ ID NO: 56;
   38) a heavy chain variable region having the amino acid sequence at least 95% identical to the amino acid sequence of SEQ ID NO: 51, and
      a light chain variable region having the amino acid sequence at least 95% identical to the amino acid sequence of SEQ ID NO: 56;
   39) a heavy chain variable region having the amino acid sequence at least 95% identical to the amino acid sequence of SEQ ID NO: 52, and
      a light chain variable region having the amino acid sequence at least 95% identical to the amino acid sequence of SEQ ID NO: 55;
   40) a heavy chain variable region having the amino acid sequence at least 95% identical to the amino acid sequence of SEQ ID NO: 50, and
      a light chain variable region having the amino acid sequence at least 95% identical to the amino acid sequence of SEQ ID NO: 56;
   41) a heavy chain variable region having the amino acid sequence at least 95% identical to the amino acid sequence of SEQ ID NO: 53, and
      a light chain variable region having the amino acid sequence at least 95% identical to the amino acid sequence of SEQ ID NO: 55;
   42) a heavy chain variable region having the amino acid sequence at least 95% identical to the amino acid sequence of SEQ ID NO: 54, and
      a light chain variable region having the amino acid sequence at least 95% identical to the amino acid sequence of SEQ ID NO: 28;
   43) a heavy chain variable region having the amino acid sequence at least 95% identical to the amino acid sequence of SEQ ID NO: 25, and
      a light chain variable region having the amino acid sequence at least 95% identical to the amino acid sequence of SEQ ID NO: 57;
   44) a heavy chain variable region having the amino acid sequence at least 95% identical to the amino acid sequence of SEQ ID NO: 25, and
      a light chain variable region having the amino acid sequence at least 95% identical to the amino acid sequence of SEQ ID NO: 28;
   45) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 79;
   46) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 80;
   47) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 88;
   48) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 101;
   49) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 102;
   50) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 81;
   51) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 82;
   52) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 83;
   53) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 84;
   54) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 85;
   55) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 86;
   56) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 87;
   57) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 89;
   58) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 90;
   59) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 91;
   60) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 92;
   61) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 93;
   62) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 94;
   63) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 95;
   64) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 96;
   65) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 97;
   66) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 98;
   67) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 99; or
   68) a heavy chain variable region having the amino acid sequence at least 95% identical to the amino acid sequence of SEQ ID NO: 33, and
      a light chain variable region having the amino acid sequence at least 95% identical to the amino acid sequence of SEQ ID NO: 100.
(7) The monoclonal antibody or its antibody fragment according to any one of (1) to (6),
   further having a heavy chain constant region having the amino acid sequence of SEQ ID NO: 11, and a light chain constant region having the amino acid sequence of SEQ ID NO: 12.
   (7-1) The monoclonal antibody or its antibody fragment according to any one of (1) to (7), which has a binding activity for Nav1.7 of 2nM or less.
   (7-2) The monoclonal antibody or its antibody fragment according to any one of (1) to (7), which has a binding activity for Nav1.7 of 1nM or less.
(8) A pharmaceutical composition comprising the monoclonal antibody or its antibody fragment according to any one of (1) to (7), (7-1), (7-2).
   (8-1) A pharmaceutical composition according to (8), which is a therapeutic and/or preventive agent for a Nav1.7 related disease.
(9) A pharmaceutical composition according to (8-1), wherein the Nav1.7 related disease is pain and/or pruritus.
(10) A polynucleotide encoding a heavy chain variable region of the antibody according to (5) or (6) and optionally encoding a heavy chain constant region of the antibody according to (7).
(11-1) A polynucleotide encoding a light chain variable region of the antibody according to (5) or (6) and optionally encoding a light chain constant region of the antibody according to (7).
(11-2) A combination of the polynucleotide according to (10) and (11-1).
(11-3) A polynucleotide encoding the monoclonal antibody or its antibody fragment according to any one of (1), (2) to (7).
(12) An expression vector comprising the polynucleotide according to (10) and/or (11-1). (12-1) A host cell comprising the polynucleotide of (11-3) or the expression vector of (12).
(13) A method for treating or preventing a Nav1.7 related disease comprising administering the monoclonal antibody or its antibody fragment according to any one of (1) to (7), (7-1), (7-2).
(14) The monoclonal antibody or its antibody fragment according to any one of (1) to (7), (7-1), (7-2), for manufacturing a therapeutic and/or preventive agent for a Nav1.7 related disease.
(15) The monoclonal antibody or its antibody fragment according to any one of (1) to (7), (7-1), (7-2), for treating or preventing a Nav1.7 related disease.
(16) Use of the monoclonal antibody or its antibody fragment according to any one of (1) to (7), (7-1), (7-2), for treating or preventing a Nav1.7 related disease.
(17) The method for treating or preventing according to (13), the antibody or its antibody fragment according to (14) or (15) or use of the monoclonal antibody or its antibody fragment according to (16), wherein the Nav1.7-related disease is pain and/or pruritus.
(18) A kit for detecting Nav1.7 comprising the monoclonal antibody or its antibody fragment according to any one of (1) to (7), (7-1), (7-2).

### [EFFECTS OF THE INVENTION]

Since a monoclonal antibody or its antibody fragment of the present invention specifically binds to Nav1.7, it may be used to detect Nav1.7 in biological samples. In addition, since a monoclonal antibody or its antibody fragment of the present invention has the activity to selectively inhibit Nav1.7, a pharmaceutical composition comprising a monoclonal antibody or its antibody fragment of the present invention is great useful for medicine, particularly for treating or preventing a Nav1.7 related disease.

### [BRIEF DESCRIPTION OF DRAWINGS]

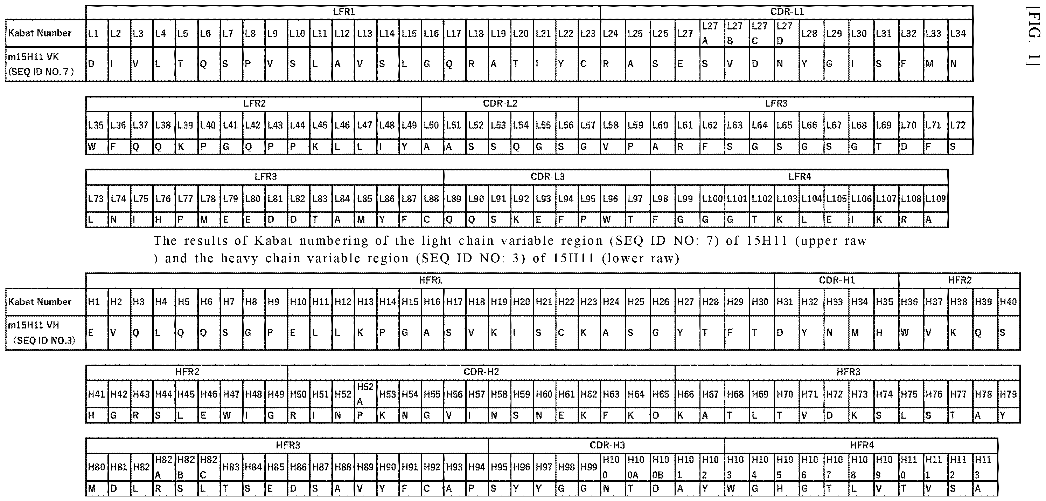

[FIG. 1] The results of Kabat numbering of the heavy chain variable region (SEQ ID NO: 3) and the light chain variable region (SEQ ID NO: 7) of 15H11 are shown. HFR1 to HFR4 mean heavy chain framework regions 1 to 4, respectively, and CDR-H1 to CDR-H3 mean heavy chain complementarity determining regions 1 to 3, respectively. LFR1 to LFR4 mean light chain framework regions 1 to 4, respectively, and CDR-L1 to CDR-L3 mean light chain complementarity determining regions 1 to 3, respectively.

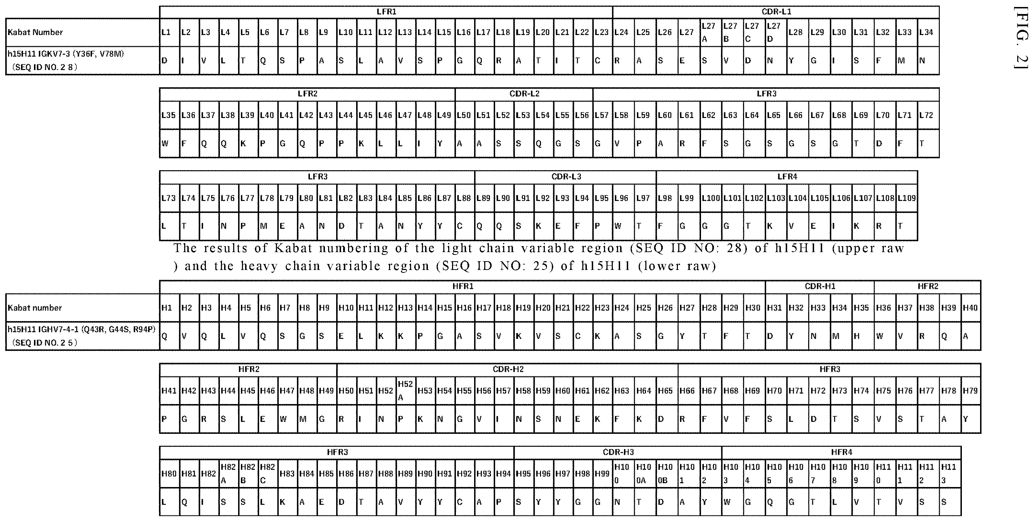

[FIG. 2] The results of Kabat numbering of the heavy chain variable regions (IGHV7-4-1 Q43R, G44S, R94P (SEQ ID NO: 25)) and the light chain variable regions (IGKV7-3 Y36F, V78M (SEQ ID NO: 28)) of the humanized antibody h15H11 are shown. HFR1 to HFR4 mean heavy chain framework regions 1 to 4, respectively, and CDR-H1 to CDR-H3 mean heavy chain complementarity determining regions 1 to 3, respectively. LFR1 to LFR4 mean light chain framework regions 1 to 4, respectively, and CDR-L1 to CDR-L3 mean light chain complementarity determining regions 1 to 3, respectively.

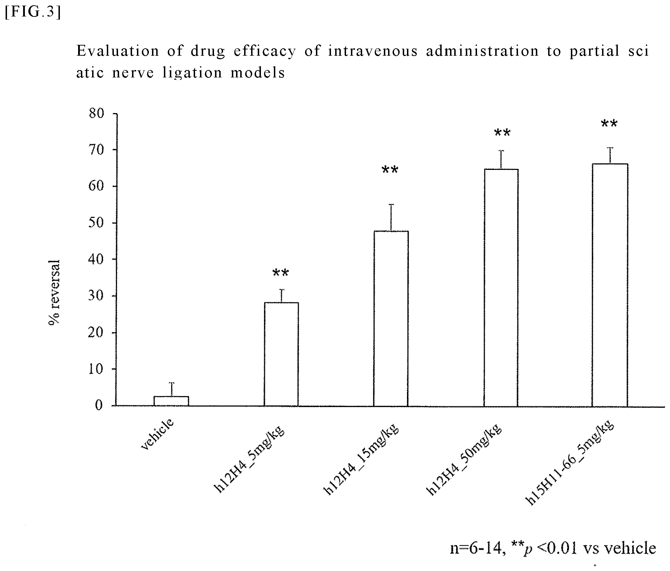

[FIG. 3] Evaluation of drug efficacy of intravenous administration to partial sciatic nerve ligation models

### [MODE FOR CARRYING OUT THE INVENTION]

Terms in the present description refer to commonly used meanings in the art, unless otherwise mentioned.

In the present invention, antibody production techniques known in the art are available. Examples include the methods described in Immunochemistry in Practice (Blackwell Scientific Publications).

Genetic engineering techniques known in the art are also available. Examples include the methods described in Molecular Cloning, A Laboratory Manual, Fourth Edition, Cold Spring Harbor Laboratory Press (2012), Current Protocols Essential Laboratory Techniques, Current Protocols (2012).

Human Nav1.7 is protein (UniProtKB/Swiss-Prot:Q15858) consisting of amino acids encoded by gene SCN9A.

A monoclonal antibody or its antibody fragment of the present invention is a monoclonal antibody or its antibody fragment having the CDRs or the heavy chain variable regions/light chain variable regions described in the present description. The antibody or its antibody fragment may be derived from any class (e.g., IgG, IgE, IgM, IgD or IgA) or subclass of immunoglobulin molecules and may be obtained from any species including mouse, rat, shark, rabbit, pig, hamster, camel, llama, goat and human. The preferable antibody or its antibody fragment is the humanized monoclonal antibody or the antibody fragment of the humanized monoclonal anitbody.

In the invention, "an antibody fragment of a monoclonal antibody" means a portion of a monoclonal antibody, wherein the fragment specifically binds to Nav1.7 and selectively inhibits Nav1.7 as well as said monoclonal antibody.

Specifically, examples include Fab (fragment of antigen binding), Fab', F(ab')₂, a single chain antibody (single chain Fv; hereinafter referred to as scFv), a disulfide stabilized antibody (disulfide stabilized Fv; hereinafter referred to as dsFv), a dimerized V-region fragment (hereinafter referred to as diabody), a peptide comprising CDR, and the like, which specifically bind to human Nav1.7 (Expert Opinion on Therapeutic Patents, vol. 6, No. 5, pp. 441-456, 1996).

Fab is an antibody fragment having an antigen-binding activity of about 50,000 molecular weight, composed of about half of the N-terminal end side of the H-chain and the entire L-chain obtained by degrading the peptide portion of the upper part of the two disulfide bonds (S-S bonds) crosslinking the two H-chains in the hinge region of IgG with an enzyme papain. Fab used in the present invention can be obtained by treating the monoclonal antibody of the present invention with papain. Fab can also be produced by inserting DNA encoding Fab of the monoclonal antibody of the present invention into a cell expression vector and introducing the obtained vector into a cell to express Fab.

Fab' is an antibody fragment having an antigen-binding activity of about 50,000 molecular weight, obtained by cleaving S-S bonds in the hinge of F(ab')₂. Fab' used in the present invention can be obtained by treating F(ab')₂ of the monoclonal antibody of the present invention with a reducing agent dithiothreitol. Fab' can also be produced by inserting DNA encoding Fab' of the monoclonal antibody of the present invention into a cell expression vector, and introducing the obtained vector into E. coli, yeast, or an animal cell to express Fab'.

F(ab')₂ is an antibody fragment having an antigen-binding activity of about 100,000 molecular weight, composed of two Fab' regions bonded at the hinge portion obtained by degrading the lower part of the two S-S bonds in the hinge region of IgG with an enzyme pepsin. F(ab')₂ used in the present invention can be obtained by treating the monoclonal antibody of the present invention with pepsin. F(ab')₂ can also be produced by inserting DNA encoding F(ab')₂ of the monoclonal antibody of the present invention into a cell expression vector, and introducing the obtained vector into E. coli, yeast, or an animal cell to express F(ab')₂.

scFv is a VH-P-VL or VL-P-VH polypeptide in which one VH and one VL are linked with an appropriate peptide linker (hereinafter referred to as P), and is an antibody fragment having antigen activity. The VH and VL contained in scFv used in the present invention may be those of the monoclonal antibody of the present invention. scFv used in the present invention can also be produced by constructing a scFv expression vector using cDNA encoding the VH and VL of the monoclonal antibody of the present invention and introducing the obtained vector into E. coli, yeast, or an animal cell to express scFv.

dsFv refers to those obtained by bonding polypeptides in which 1 amino acid residue in VH and VL is substituted with a cysteine residue, respectively, via an S-S bond. The amino acid residues to be substituted with cysteine residues can be selected based on stereostructural predictions of the antibody according to the method shown by Reiter et al. (Protein Engineering, 7, 697 (1994)). The VH or VL contained in the dsFv used in the present invention may be those of the monoclonal antibody of the present invention. dsFv used in the present invention can also be produced by constructing a dsFv expression vector by inserting cDNA encoding the VH and VL of the monoclonal antibody of the present invention into an appropriate expression vector and introducing the obtained vector into E. coli, yeast, or an animal cell to express dsFv.

The diabody is an antibody fragment in which scFvs having the same or different antigen-binding specificity form a dimer, and is an antibody fragment having divalent antigen-binding activity for the same antigen or two different specific antigen-binding activities for different antigens. For example, a divalent diabody that specifically reacts to the monoclonal antibody of the present invention can be produced by using cDNA encoding the VH and VL of the monoclonal antibody of the present invention to construct a DNA encoding scFv having a peptide linker of 3 to 10 residues, inserting the DNA into a cell expression vector, introducing the obtained expression vector into E. coli, yeast, or an animal cell to express the diabody.

A peptide comprising a CDR is composed of at least one or more regions of a CDR of VH or VL. The plurality of CDRs can be bound directly or via an appropriate peptide linker. The peptide comprising a CDR used in the present invention can be produced by constructing a CDR-encoding DNA using cDNA encoding the VH and VL of the monoclonal antibody of the present invention, inserting the DNA into an animal cell expression vector, and introducing the obtained vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

The monoclonal antibody of the present invention includes an antibody derivative in which a radioactive isotope, a low-molecular or a high-molecular drug, a protein, or an antibody drug different from the monoclonal antibody of the present invention etc. is chemically or genetically engineered to a monoclonal antibody specifically recognizing and binding to the amino acid sequence of human Nav1.7 or its three-dimensional structure.

The monoclonal antibody or its antibody fragment of the present invention is characterized by specific binding to Nav1.7. An example of procedures for measuring the specific binding ability to Nav1.7 is shown as follows.

The specific binding of Nav1.7 can be evaluated as binding activity by measuring whether two molecules specifically bind. For example, in addition to methods well known in the art, competitive ELISA methods described in Example 5, surface plasmon resonance, etc. may be used.

The effective concentration of the antibody that exhibits a 50% inhibition rate of Nav1.7 binding competitive inhibitory activity by competitive ELISA is defined as IC50. IC50 is often used as an indicator of antibody binding activity. The IC50 value of the monoclonal antibody of the present invention is preferably 2nM or less, more preferably 1nM or less, particularly preferably 0.6nM or less.

The binding activity of Nav1.7 by surface plasmon resonance is defined as the equilibrium dissociation constant (KD). For example, a smaller KD represents tighter binding. The KD of the monoclonal antibody or its antibody fragment of the present invention is preferably about 2nM or less, more preferably about 1nM or less.

For example, an antibody with a binding activity of 2nM or less for Nav1.7 means a Nav1.7 monoclonal antibody with an IC50 and/or KD of 2nM or less.

However, isolated antibodies that specifically bind Nav1.7 may exhibit cross-reactivity to other antigens, such as Nav1.7 molecules from other species. Nevertheless, further, a multispecific antibody that binds hNav1.7 and one or more additional antigens, or a multispecific antibody that binds two kinds of different regions of hNav1.7 (e.g., the E1 extracellular loop of domain C and the E3 extracellular loop of domain C) is considered an antibody that "specifically binds" to hNav1.7.

The monoclonal antibody or its antibody fragment of the present invention is characterized by inhibiting Nav1.7. An example of measurement procedure of the inhibitory ability of Nav1.7 is shown below.

Cells stably expressing Nav1.7 are constructed by transfecting FreeStyle 293 cells (Thermo Fisher Scientific) with pcDNA3.1 (manufactured by Invitrogen) cloned from DNA encoding Nav1.7. By performing manual patch clamp using these cells according to the method described below, the Nav1.7-specific inhibition of the antibody can be evaluated. Glass pieces coated with poly-L-lysine are arranged in a 35 mm dish, and cells stably expressing Nav1.7 suspended in DMEM containing 10% FBS (manufactured by SIGMA) are seeded (4 × 104 cells/dish). On the day after seeding, the glass piece is transferred to a measurement chamber to form a whole cell, to be fixed at -70 mV, an antibody is treated while given a 10 msec square wave at 0.1 Hz and measured before and after antibody treatment (>2 min treatment).

The monoclonal antibody of the present invention may be produced by using the CDRs or the heavy and light chain variable regions described in the present description, according to the routine methods in the art.

The monoclonal antibody of the present invention further includes a humanized monoclonal antibody. Because a humanized antibody has reduced immunogenicity (antigenicity) in the human body, it's useful when administrated into humans for the purpose of therapy and the like. "Immunogenicity is low" means, for example, that the administered monoclonal antibody of the present invention does not induce an immune response by an organism in sufficient time to achieve a therapeutic effect. The level of immunogenicity in humans can be predicted with T cell epitope prediction programs. For example, Epibase (Lonza), iTope/TCED (Antitope), EpiMatrix (EpiVax), etc. are used as T cell epitope prediction programs.

A humanized monoclonal antibody is an antibody in which a complementarity determining region (CDR) of a non-human mammal antibody, such as a mouse antibody, is implanted into a framework region (FR) of a human antibody. The FR of a humanized monoclonal antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized monoclonal antibody form an appropriate antigen binding site (Sato, K. et al., Cancer Res. 1993, vol. 53, p. 851). The percentage of amino acids of the FR to be substituted is from 0 to 15%, preferably from 0 to 5%, of the total FR region.

It should be noted that the humanized monoclonal antibody of the present invention uses a constant region of a human antibody. Preferred examples of the constant region of a human antibody include Cγ, such as Cγ1, Cγ2, Cγ3, or Cγ4, as the heavy chain, and Cκ and Cλ, as the light chain. The constant region of the human antibody may be modified to improve stability of the antibody or its production. The human antibody used in producing the humanized antibody may be any isotype of human antibody, such as IgG, IgM, IgA, IgE or IgD, and, in the present invention, IgG is preferably used, and IgG1 or IgG4 is further preferably used. When selecting IgG1, it is preferable to have an amino acid mutation in the heavy chain constant region to reduce effector function. Preferred amino acid mutations include mutations such as N297G/A, L234A, L235A, D265A, and P329G at amino acid residues N297, L234, L235, D265, and/or P329 in Kabat numbering. IgG1 may contain any one or a combination of these mutations.

The humanized monoclonal antibody of the present invention preferably has a light chain constant region of the amino acid sequence of SEQ ID NO: 12 and a heavy chain constant region of the amino acid sequence of SEQ ID NO: 11. However, in SEQ ID NO: 11, the C-terminal lysine or the C-terminal two amino acid residues (glycine-lysine) may or may not be present.

The humanized monoclonal antibody can be made by general production methods (see, e.g., Example 4 below, WO 95/14041, WO 96/02576). Specifically, at first, a DNA sequence encoding a variable region designed to link a CDR of a mouse antibody to a FR of a human antibody is synthesized by PCR method from several oligonucleotides which are made to have moieties overlapping the terminal ends (see, WO 98/13388). The obtained DNA is linked to a DNA encoding a constant region of a human antibody, and then incorporated into an expression vector. Alternatively, a DNA encoding a variable region of an antibody may be incorporated into an expression vector comprising a DNA of a constant region of an antibody. To produce an antibody used in the present invention, the antibody gene is incorporated into an expression vector such that it is expressed under the control of an expression control region, e.g., an enhancer/promoter. This expression vector can then be used to transform a host cell and express the antibody.

Examples of the above host cell of the transformant include a vertebrate cell such as a COS cell or a CHO cell, a prokaryotic cell, and a yeast. The transformant can be cultured according to a method well known to those skilled in the art, and the monoclonal antibody of the present invention is produced intracellularly or extracellularly of the transformant. The medium used for the culture can be selected as appropriate depending on the adopted host cell from various types of media commonly used. For example, in the case of a COS cell, examples of the medium include a medium such as RPMI-1640 medium or Dulbecco's Modified Eagle Minimum Essential Medium (DMEM) supplemented with serum components such as bovine fetal serum (FBS), as necessary. The culture temperature during culture of the transformant may be any temperature that does not significantly reduce protein synthesis capacity in the cell, and is preferably 32 to 42°C, most preferably 37°C. As necessary, the transformant can be cultured in an atmosphere containing 1 to 10% (v/v) of carbon dioxide.

Fractions comprising the monoclonal antibody of the present invention produced intracellularly or extracellularly of the transformant as described above can be separated and purified by various known separation methods utilizing the physical and chemical properties or the like of the proteins. Specific examples of such methods include usual treatment with a protein precipitant, ultrafiltration, various chromatography such as molecular sieve chromatography (gel filtration), adsorption chromatography, ion exchange chromatography, affinity chromatography, or high-performance liquid chromatography (HPLC), a dialysis method, and a combination of these. With the methods, the monoclonal antibody of the present invention can be readily produced in high yield and high purity.

The monoclonal antibody or its active fragment of the present invention may be further modified by various molecules such as polyethylene glycol (PEG), radioactive materials, toxins, and the like. As the method for modifying the antibody, known methods in the art can be used.

Furthermore, other proteins may be fused to the N- or C-terminal end of the monoclonal antibody of the present invention (Clinical Cancer Research, 2004, 10, 1274-1281). The protein to be fused can be appropriately selected by those skilled in the art.

A pharmaceutical composition comprising the monoclonal antibody or its antibody fragment of the present invention (a pharmaceutical composition of the present invention) can be administrated systemically or topically, orally or parentally. Examples of the parenteral administration include intravenous injection such as drip infusion, intramuscular injection, intraperitoneal injection, subcutaneous injection, intranasal administration, and inhalation.

A pharmaceutical composition of the present invention is highly useful as a medicine for the treatment and/or prevention of Nav1.7 related diseases.

Nav1.7 related diseases include pain, pruritus, neurogenic inflammations, cough, and the like.

"Pain" includes acute pains, chronic pains, neuropathic pains, inflammatory pain, arthritis, bone arthritis, migraine, cluster headache syndrome, trigeminal neuralgia, herpetic neuralgia, systemic neuralgia, neurodegenerative diseases, movement disorders, neuroendocrine disorders, ataxia, sepsis, visceral pain, acute gout, post-herpetic neuralgia, diabetic neuropathy, sciatica, back pain, head or neck pain, severe pain or intractable pain, sudden pain, pain after surgery, erythromelalgia genetic, dental pain, rhinitis, cancer pain and bladder disorder.

"Pruritus" includes acute pruritus, chronic pruritus, histamine dependent pruritus and histamine independent pruritus.

"Neurogenic inflammation" may be associated with asthma, arthritis, eczema, headache, migraine, or psoriasis, or a combination thereof.

"Cough" includes pathological or chronic cough.

Effective dose of the pharmaceutical composition of the present invention is selected in the range of 0.01 mg to 100 mg per 1 kg of body weight per one time. Alternatively, a dose of 5 to 5000 mg, preferably a dose of 10 to 500 mg per a patient may be selected. However, a dose of the pharmaceutical composition comprising the monoclonal antibody or its antibody fragment of the present invention is not limited to these doses. Also, administering duration may be also appropriately selected depending on the age and symptom of the patient. The pharmaceutical composition of the present invention may also comprise a pharmaceutically acceptable carrier or additive as well depending on the route of administration. Examples of such a carrier and an additive include water, pharmaceutically acceptable organic solvent, collagen, polyvinyl alcohol, polyvinylpyrrolidone, sodium alginate, water-soluble dextran, pectin, methyl cellulose, ethyl cellulose, casein, diglycerin, propylene glycol, polyethylene glycol, Vaseline, human serum albumin (HSA), mannitol, sorbitol, lactose, and surfactants permitted as a pharmaceutical additive. An additive to be used is selected as appropriate or in combination from the above depending on the dose form, but it is not limited thereto.

The monoclonal antibody or its antibody fragment of the present invention may be administered as a concomitant drug in combination with other active ingredients or drugs comprising other active ingredients. Other active ingredients include, for example, opioids, COX-2 inhibitors, local anesthetics, NMDA modulators, cannabinoid receptor agonists, P2X family modulators, VR1 antagonists, substance P antagonists, antiepileptic drugs (such as gabapentin, pregabalin or topiramate etc.) , tricyclic antidepressants (such as amitriptyline etc.), celecoxib, cytokine inhibitors or antagonists (such as antagonists against IL-6, IL-6R, IL-18 or IL-18R etc.), Nav1.8 inhibitors, Nav1.9 inhibitors agents, NGF inhibitors, Nav1.7 inhibitors or antagonists, other antibodies specific for Nav1.7, polypeptide antagonists to Nav1.7, siRNA, antisense molecules, low molecular weight drugs, protein/polypeptide inhibition agents, etc.

The concomitant drug of the monoclonal antibody or its antibody fragment of the present invention and other active ingredients may be administered in the form of a combination drug comprising both components in one preparation or may be administered in the form of a separate preparation (the drug comprising the pharmaceutical composition of the present invention and the other active ingredients). Administration in separate formulations includes simultaneous administration and staggered administration. In addition, for staggered administration, the pharmaceutical composition of the present invention may be administered first and the other drug be later, or the other drug may be administered first and the pharmaceutical composition of the present invention be later, and the respective administration methods may be the same or different.

The present invention comprises a polynucleotide encoding a heavy chain variable region and/or light chain variable region of a monoclonal antibody of the present invention. A polynucleotide encoding a heavy chain variable region of a monoclonal antibody of the present invention may further encode a heavy chain constant region. A polynucleotide encoding a light chain variable region of a monoclonal antibody of the present invention may further encodes a light chain constant region. The present invention also includes expression vectors comprising at least one of these polynucleotides.

The polynucleotide is not particularly limited as long as it encodes a light chain variable region or a heavy chain variable region of the monoclonal antibody of the present invention, and it is a polymer consisting of nucleotides such as a plurality of deoxyribonucleic acids (DNA) or ribonucleic acids (RNA). It may contain a non-natural nucleotide. The polynucleotide of the present invention can be used for producing antibodies by genetically engineered methods. The polynucleotide of the present invention can also be used as a probe for screening an antibody having the same function as the monoclonal antibody of the present invention. That is, a polynucleotide encoding the monoclonal antibody of the present invention or a portion thereof can be used as a probe in techniques such as hybridization, gene amplification techniques (e.g., PCR) or the like to obtain a DNA that hybridizes with the polynucleotide under stringent conditions and encodes an antibody having equivalent activity to the monoclonal antibody of the present invention. Such DNA is also included in the polynucleotide of the present invention.

Hybridization techniques (Sambrook, J et al., Molecular Cloning 2nd ed., 9.47-9.58, Cold Spring Harbor Lab. press, 1989) are techniques well known to those skilled in the art. Examples of conditions for hybridization include low stringent conditions. A low stringent condition is, for example, a condition of 42°C, 0.1 × SSC, 0.1% SDS, preferably a condition of 50°C, 0.1 × SSC, 0.1% SDS in washing after hybridization. Examples of more preferred hybridization conditions include high stringent conditions. A high stringent condition is, for example, a condition of 65°C, 5 × SSC and 0.1% SDS. In these conditions, it can be expected that polynucleotides having high homology are efficiently obtained as the temperature increases. However, multiple factors such as temperature and salt concentration are considered to affect the stringency of hybridization. Those skilled in the art can achieve the same stringency by selecting these factors as appropriate.

Antibodies that are functionally equivalent to the monoclonal antibody of the present invention, encoded by the polynucleotides obtained by these hybridization and gene amplification techniques, typically have high homology with the antibody in amino acid sequence. The monoclonal antibody of the present invention also includes an antibody that is functionally equivalent to the monoclonal antibody of the present invention and has high homology with the antibody in amino acid sequence. High homology usually refers to at least 75% or more identity, preferably 85% or more identity, further preferably 95% or more identity in amino acid level. The homology of a polypeptide can be determined according to the algorithm described in the document (Wilbur, W. J. and Lipman, D. J. Proc. Natl. Acad. Sci. USA (1983) 80, 726-730).

The monoclonal antibody or an antibody fragment thereof of the present invention specifically binds to Nav1.7 and can thus be used for detecting Nav1.7 in a biological sample. Examples of the biological sample include blood, plasma, serum, urine, an organ, a tissue, bone marrow, and a lymph node. Accordingly, a kit comprising the monoclonal antibody of the present invention is available as a kit for detecting Nav1.7. The kit comprises the monoclonal antibody or an antibody fragment thereof of the present invention, and may further comprise a secondary antibody for labelling, a substrate necessary for detection of the labelling, a carrier, a wash buffer, a sample dilution, an enzyme substrate, a reaction stop solution, a Nav1.7 protein as a purified standard substance, an instruction for use, and the like.

A monoclonal antibody or its antibody fragment of the present invention has any or all of the following excellent characteristics.
a) Having strong binding activity to the E3 extracellular loop C-terminal region of domain C of Nav1.7.
b) Having strong drug efficacy for pain suppression.
c) Low immunogenicity to humans after humanization.
d) Long duration of drug efficacy for pain suppression after humanization.
e) Extremely high selectivity for peptides corresponding to epitope site of E3 extracellular loop C-terminal region of other subtypes, hNav1.1, hNav1.2, hNav1.3, hNav1.4, hNav1.5, hNav1.6, hNav1.8 and hNav1.9.

### [EXAMPLES]

Hereinafter, the present invention will be specifically described with reference to Examples. However, the present invention is not limited by Examples given below.

### Example 1: Preparation of an Nav1.7 antibody

A peptide (1424-QPKYEYSL (SEQ ID NO: 1)-1431; hCE3C peptides) corresponding to domain C and E3 extracellular loop C terminal regions of human Nav1.7 (UniProtKB/Swiss-Prot:Q15858) was selected as an antigen.

A peptide (CQPKYEYSL (SEQ ID NO: 2); Cys-hCE3C) to which a Cys residue was added at the N-terminal end of a hCE3C peptide (manufactured by Toray Corporation) was synthesized, was bound to maleimidated giant keyhole limpets hemocyanin (maleimidated KLH, manufactured by Thermo Scientific Corporation) to prepare an immunogen. This peptide-KLH complex was immunized with Freund's complete adjuvant together to A/J Jms Slc female mice. Thereafter, the mice were additionally immunized five times together with Freund's incomplete adjuvant.

Three days after the final immunization, the spleen was removed, splenocytes and mouse myeloma cells (p3x6363-Ag8., Tokyo Oncology Institute) were fused by the PEG-method, and selection was made in a medium containing hypoxanthine, aminopterin and thymidine. Human Nav1.7-binding antibodies were identified by performing ELISA against immunogenic peptides using hybridoma culture supernatants. Specifically, hybridoma culture supernatant was added to an anti-mouse IgG antibody immobilized plate, washed twice with ELISA wash buffer, and biotin-labeled immunogenic peptide (Cys-hCE3C) and Streptavidin-HRP (manufactured by PIERCE) was added and reacted overnight at 4°C. After washing twice with ELISA wash buffer and developing color by adding TMB-Substrate Chromogen (manufactured by Thermo Fisher Scientific), the reaction was stopped with an equal volume of 0.5N sulfuric acid, and the absorbance at 450 nm was measured. Hybridomas that showed strong binding signals in ELISA were cloned and established.

### Example 2: Determination of antibody sequences

The amino acid sequences of the heavy and light chain variable regions of the mouse monoclonal antibody 15H11, were determined from hybridoma cells of established clones by conventional methods (Table 1).

**[Table 1]**

| mAb | Variable regions | SEQID | | SEQID | Variable regions | SEQID | | SEQID |
|---|---|---|---|---|---|---|---|---|
| 15H11 | Heavy chain | 3 | CDR 1 | 4 | Light chain | 7 | CDR 1 | 8 |
| | | | CDR 2 | 5 | | | CDR 2 | 9 |
| | | | CDR 3 | 6 | | | CDR 3 | 10 |

### Example 3: Alignment of antibody sequences

The amino acid sequences of the heavy chains and light chains of 15H11 were subjected to Kabat numbering using the antibody-sequence analysis software abYsis (FIG. 1).

### Example 4: Humanization of antibody

15H11 was humanized by the following method. Human germline acceptor sequences, similar to the V gene domain sequences of the heavy chains and light chains of 15H11, were searched and selected by sequence analysis software AbYsis. Regarding the J-chain regions, multiple sequences with high homologous sequences to DNA sequences of the mouse antibody were searched by IMGT (http://www.imgt.org/) and were determined as human framework sequences. To this human framework sequence, the mouse antibody heavy chain CDR1, CDR2, and CDR3 and the mouse antibody light chain CDR1, CDR2, and CDR3 as defined by Kabat numbering (Wu, T. T. and Kabat, E. A., J Exp. Med. Aug1;132(2):211-50. (1970)) were implanted to design the humanized antibody sequences listed in Table 2 (CDR graft body). As the constant region of h15H11, hIgG4Pro (SEQ ID NO: 11) was used for the heavy chain and hIgK (SEQ ID NO: 12) was used for the light chain. Humanized antibody sequences (CDR graft body) are expressed in the culture supernatant using Expi293 cells, and regarding the antibodies present in the supernatant, the titer (ratio) against the epitope peptide per unit IgG amount was calculated by the method described below and shown in Table 2.

The amount of IgG in the supernatant was determined by the following method. To evaluate the amount of IgG in the supernatant, the culture supernatant was added to an anti-human IgG antibody (manufactured by Jackson) immobilized plate, after reacting at room temperature for 3 hours, washed twice with ELISA wash buffer, and HRP-labeled anti-human IgG antibody (manufactured by Jackson) was reacted at 4° C overnight. After washing twice with ELISA wash buffer and developing color by adding TMB-Substrate Chromogen (manufactured by Thermo Fisher Scientific), the reaction was stopped with an equal volume of 0.5N sulfuric acid, and the absorbance at 450 nm was measured to calculate (A) the dilution factor of the culture supernatant at which the OD was 1.0.

The titer of IgG in the supernatant against the epitope peptide was determined by the following method. In order to evaluate the titer of IgG in the supernatant against epitope peptides, the culture supernatant was added to an anti-human IgG antibody (manufactured by Jackson) immobilized plate, reacted for 3 hours at room temperature, and then washed twice with ELISA wash buffer, and a biotin-labeled epitope peptide (Cys-CE3C long: SEQ ID NO: 13) and Streptavidin-HRP (manufactured by PIERCE) were added and reacted overnight at 4° C. After washing twice with ELISA wash buffer and developing color by adding TMB-Substrate Chromogen (manufactured by Thermo Fisher Scientific), the reaction was stopped with an equal volume of 0.5N sulfuric acid, the absorbance at 450 nm was measured to calculate (B) the dilution ratio of the culture supernatant at which the OD was 1.0.

The titer (ratio) against the epitope peptide (Cys-CE3C long: SEQ ID NO: 13) per unit IgG amount was calculated as (B)/(A). From the results in Table 2, it was found that IGKV7-3 is suitable as the light chain human framework sequence of humanized 15H11, and IGHV7-4-1 (R94P), IGHV1-2 (R94P), IGHV1-18 (R94P) and that IGHV1-3 (R94P) are suitable as the heavy chain human framework sequences.

**[Table 2]**

| Light chain | | Heavy chain | | |
|---|---|---|---|---|
| Framework | Variable regions SEQ ID. | Framework | Variable regions SEQ ID. | (B)/(A) |
| IGKV7-3 | 14 | IGHV 1-2 | 19 | 0.04 |
| | | IGHV 1-2 (R94P) | 20 | 0.22 |
| | | IGHV 1-3 (R94P) | 21 | 0.21 |
| | | IGHV 1-18 (R94P) | 22 | 0.24 |
| | | IGHV 1-46 (R94P) | 23 | 0.18 |
| | | IGHV 7-4-1 (R94P) | 24 | 0.26 |
| IGKV1-39 | 15 | IGHV 1-2 | 19 | 0.03 |
| | | IGHV 1-2 (R94P) | 20 | 0.14 |
| | | IGHV 1-3 (R94P) | 21 | 0.12 |
| | | IGHV 1-18 (R94P) | 22 | 0.13 |
| | | IGHV 1-46 (R94P) | 23 | 0.09 |
| | | IGHV 7-4-1 (R94P) | 24 | 0.16 |
| IGKV3-11 | 16 | IGHV 1-2 | 19 | 0.05 |
| | | IGHV 1-2 (R94P) | 20 | 0.15 |
| | | IGHV 1-3 (R94P) | 21 | 0.12 |
| | | IGHV 1-18 (R94P) | 22 | 0.15 |
| | | IGHV 1-46 (R94P) | 23 | 0.12 |
| | | IGHV 7-4-1 (R94P) | 24 | 0.18 |
| IGKV3-20 | 17 | IGHV 1-2 | 19 | 0.04 |
| | | IGHV 1-2 (R94P) | 20 | 0.12 |
| | | IGHV 1-3 (R94P) | 21 | 0.12 |
| | | IGHV 1-18 (R94P) | 22 | 0.13 |
| | | IGHV 1-46 (R94P) | 23 | 0.09 |
| | | IGHV 7-4-1 (R94P) | 24 | 0.15 |
| IGKV4-1 | 18 | IGHV 1-2 | 19 | 0.03 |
| | | IGHV 1-2 (R94P) | 20 | 0.18 |
| | | IGHV 1-3 (R94P) | 21 | 0.15 |
| | | IGHV 1-18 (R94P) | 22 | 0.13 |
| | | IGHV 1-46 (R94P) | 23 | 0.13 |
| | | IGHV 7-4-1 (R94P) | 24 | 0.21 |

Furthermore, by introducing mutations (backmutation) into the human framework sequence of the humanized antibody sequence (CDR graft body), the recovery of the affinity for the epitope peptide is tried so that it has almost the same affinity as the mouse antibody 15H11. Using IGKV7-3 and IGHV7-4-1 (R94P), mutants with back mutations introduced at the positions shown in Table 3 were produced regarding the light chain, the affinity for the epitope peptide was evaluated by the method shown in Example 5, and Y36F (meaning that the L36 amino acid site according to Kabat numbering was mutated from Y to F, and the same applies hereinafter) showed clear affinity improvement, while T72S, T74N, and V78M showed weak affinity improvement trend. In Table 3, ○ means clear affinity improvement, △ means weak affinity improvement, and × means that no affinity improvement is observed.

**[Table 3]**

| Light chain mutation | Affinity improvement trend |
|---|---|
| A9V | × |
| T22Y | × |
| Y36F | ○ |
| T72S | Δ |
| T74N | Δ |
| N76H | × |
| V78M | Δ |
| A80E | × |
| N81D | × |
| N85M | × |
| Y87F | × |

| | |
|---|---|
| 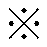 Amino acid positions are according to Kabat numbering. | |

Also, mutants in which humanized mutations were introduced into the positions shown in Table 4 were produced regarding the light chain using the mouse antibody 15H11, F36Y showed clear affinity decline, and M85N showed a weak affinity decline. In Table 4, ○ means clear affinity decline, △ means weak affinity decline, and × means that no affinity decline is observed.

**[Table 4]**

| Light chain mutation | Affinity decline trend |
|---|---|
| V9A | × |
| Y22T | × |
| F36Y | ○ |
| S72T | × |
| N74T | × |
| H76N | × |
| M78V | × |
| E80A | × |
| D81N | × |
| M85N | Δ |
| F87Y | × |

| | |
|---|---|
| 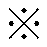 Amino acid positions are according to Kabat numbering. | |

Furthermore, mutants in which back mutations were introduced into the positions shown in Table 5 were produced regarding the heavy chain using IGKV7-3 and IGHV1-2 (R94P), Q43R and G44S showed clear affinity improvement. In Table 5, ○ means clear affinity improvement, and × means that no affinity improvement is observed.

**[Table 5]**

| Heavy chain mutation | Affinity improvement trend |
|---|---|
| V5Q, A9P | × |
| V11L, K12L | × |
| A40S, P41H | × |
| Q43R, G44S | ○ |
| T73K | × |

| | |
|---|---|
| 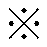 Amino acid positions are according to Kabat numbering. | |

From the above results, Y36F, V78M, N85M, T72S, and T74N for the light chain and Q43R, G44S, and R94P for the heavy chain were selected, mutants by combining them as shown in Table 6 were produced, and the affinity for the epitope peptide (Cys-CE3C long: SEQ ID NO: 13) was evaluated. As a result of calculating the ratio of the affinity of each mutant to the affinity of mouse 15H11 as Fold change (FC; affinity of mutant (IC50)/affinity of mouse 15H11 (IC50)), Q43R, G44S, and R94P were identified as heavy chain back mutations necessary to obtain affinity close to that of mouse 15H11, and Y36F and V78M were identified as light chain back mutations. As a result of the evaluation using an in vivo drug efficacy evaluation model regarding h15H11 (IGHV7-4-1 Q43R, G44S, R94P/IGKV7-3 Y36F, V78M), h15H11 (IGHV1-2 Q43R, G44S, R94P/IGKV7-3 Y36F, V78M), both showed strong pharmacological effects, namely pain suppressing effects. Note that these pharmacological effects can be measured by the method described in Example 7 of WO2019/230856.

**[Table 6]**

| Heavy chain variable regions | | | Light chain variable regions | | | |
|---|---|---|---|---|---|---|
| Framework | Mutations | SEQ ID. | Framework | Mutations | SEQ ID. | FC |
| IGHV7-4 - 1 | Q43R, G44S, R94P | 25 | IGKV7-3 | Y36F, V78M, N85M | 27 | 1.8 |
| | | | | Y36F, V78M | 28 | 1.8 |
| | | | | Y36F, N85M | 29 | 3.2 |
| | | | | Y36F, T72S, T74N | 30 | 2.9 |
| IGHV 1-2 | Q43R, G44S, R94P | 26 | | Y36F, V78M, N85M | 27 | 1.5 |
| | | | | Y36F, V78M | 28 | 1.5 |
| | | | | Y36F, N85M | 29 | 1.9 |
| | | | | Y36F, T72S, T74N | 30 | 2.0 |
| | | | | Y36F | 31 | 1.9 |
| mouse | | 3 | mouse | | 7 | 1 |

| | | | | | | |
|---|---|---|---|---|---|---|
| 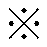 Amino acid positions are according to Kabat numbering. | | | | | | |

### Example 5: Affinity evaluation

In Examples 4, 6, and 7 of the present description, the antibody affinity for the epitope peptide (Cys-CE3C long: SEQ ID NO: 13) was measured by the competitive ELISA method shown below.

A diluted solution of the obtained recombinant humanized antibody, a biotin-labeled immunogen peptide, and Streptavidin-HRP (manufactured by PIERCE) were added to an anti-human IgG antibody-immobilized plate, and reacted overnight at 4° C. After washing twice with ELISA wash buffer and developing color by adding TMB-Substrate Chromogen (manufactured by Thermo Fisher Scientific), the reaction was stopped with an equal volume of 0.5N sulfuric acid, the absorbance at 450 nm was measured to calculate the dilution ratio of each recombinant human antibody which resulted in an OD of 1.1. Next, a recombinant human antibody at the dilution rate, a biotin-labeled immunogen peptide, and Streptavidin-HRP (manufactured by PIERCE) were added. At the same time, a dilution series of unlabeled immunogenic peptide was added and reacted overnight at 4° C. After washing twice with ELISA wash buffer, TMB-Substrate Chromogen (manufactured by Thermo Fisher Scientific) was added to develop color, the reaction was stopped with an equal amount of 0.5N sulfuric acid, and the absorbance at 450 nm was measured. The concentration of the unlabeled peptide which attenuated the signal of the biotin-labeled peptide by half was defined as the affinity (IC50).

### Example 6: Identification of critical amino acids for binding of humanized 15H11

About humanized antibody h15H11 (heavy chain variable region: IGHV7-4-1 Q43R, G44S, R94P (SEQ ID NO: 25)/light chain variable region: IGKV7-3 Y36F, V78M (SEQ ID NO: 28)) produced in Example 4, Kabat numbering and CDR definition were performed using the antibody sequence analysis software abYsis. The results are shown in FIG 2. In order to identify critical amino acids for binding of humanized 15H11, using h15H11 (heavy chain variable region: IGHV7-4-1 Q43R, G44S, R94P (SEQ ID NO: 25)/light chain variable region: IGKV7-3 Y36F, V78M (SEQ ID NO: 28) as the basic skeleton, mutants were created in which point mutations were introduced into each CDR sequence of 15H11 (SEQ ID NOs: 4 to 6, SEQ ID NOs: 8 to 10, hereinafter referred to as the wild type CDR). The affinity was calculated by the method described in Example 5, and the ratio of the affinity (IC50 of CDR variant/IC50 of wild type CDR) of each CDR variant (IC50) to the affinity of the wild type of CDR (IC50) was calculated as Fold change (FC). The results for the heavy chain are shown in Table 7, and the results for the light chain are shown in Table 8. n. d. means no detectable. As a result, when the amino acids of M34 and A101 in the heavy chain and Q90, P95, and W96 in the light chain were substituted with other amino acids, the binding activity to this peptide became below the detection limit. From this, Q90, P95, and W96 of the light chain and M34 and A101 of the heavy chain are considered to be particularly important amino acids for binding. Also, when the heavy chain N33, N52, I57, F63, S95, Y96, G98 and the light chain F32, N34, Q89, S91, F94 amino acids are replaced with other amino acids, the affinity decreased by 1.5 times or more. From this, it is considered that F32, N34, Q89, S91, F94 of the light chain and N33, N52, I57, F63, S95, Y96, G98 of the heavy chain are important amino acids for binding.

**[Table 7]**

| Heavy chain mutants | | FC |
|---|---|---|
| CDR1 | D31E | 0.92 |
| | Y32F | 1.14 |
| | N33Q | 1.72 |
| | M34L | n.d |
| CDR2 | R50K | 0.91 |
| | I51L | 1.40 |
| | N52Q | 1.70 |
| | P52aG | 1.07 |
| | K53R | 1.03 |
| | N54Q | 0.96 |
| | N54D | 1.09 |
| | V56L | 0.92 |
| | I57L | 1.69 |
| | N58Q | 0.94 |
| | S59T | 0.80 |
| | N60Q | 1.46 |
| | E61D | 0.96 |
| | K62R | 1.14 |
| | F63L | 1.58 |
| | K64R | 1.12 |
| | D65E | 1.03 |
| CDR3 | S95T | 2.45 |
| | Y96R | 1.59 |
| | Y97F | 0.92 |
| | G98A | 1.92 |
| | G99A | 1.34 |
| | N100Q | 1.01 |
| | T100aS | 0.81 |
| | D100bE | 1.17 |
| | A101V | n.d |
| | Y102F | 1.20 |

| | | |
|---|---|---|
| 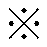 Amino acid positions are according to Kabat numbering. | | |

**[Table 8]**

| Light chain mutants | | FC |
|---|---|---|
| CDR1 | R24K | 1.07 |
| | A25V | 1.21 |
| | S26T | 1.11 |
| | E27D | 0.92 |
| | S27aT | 1.08 |
| | V27bL | 0.82 |
| | D27cE | 0.80 |
| | N27dQ | 1.04 |
| | Y28F | 0.88 |
| | G29A | 1.44 |
| | S31T | 1.14 |
| | F32L | 1.76 |
| | M33L | 0.88 |
| | N34Q | 1.80 |
| CDR2 | A50V | 1.36 |
| | A51V | 0.85 |
| | S52T | 0.90 |
| | S53T | 0.96 |
| | Q54N | 0.91 |
| | G55A | 0.90 |
| | S56T | 0.88 |
| CDR3 | Q89N | 1.75 |
| | Q90N | n.d |
| | S91T | 2.01 |
| | K92R | 0.90 |
| | E93D | 1.21 |
| | F94L | 2.46 |
| | P95G | n.d |
| | W96Y | n.d |
| | T97S | 1.17 |

| | | |
|---|---|---|
| 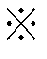 Amino acid positions are according to Kabat numbering. | | |

### Example 7 Improvement of antibody affinity

In order to produce affinity improvement antibodies of 15H11, in Example 6, based on amino acids showing FC > 1.5 and being identified to be contributing to improving the affinity of antibodies and amino acids showing FC < 1 and having possibilities to contribute to improving the affinity antibodies, various mutants having amino acid mutations in any of the CDRs were produced by using the humanized antibody h15H11 of Example 4 (heavy chain variable region: IGHV7-4-1 Q43R, G44S, R94P (SEQ ID NO: 25)/light chain variable region: IGKV7- 3 Y36F, V78M (SEQ ID NO: 28)) as a wild-type antibody (h15H11-wt). Affinity evaluation was performed by the method described in Example 5, and 43 types of affinity improvement antibodies (h15H11-1 to h15H11-43) shown in Tables 9 and 10 were obtained.

**[Table 9]**

| | Light chain variable regions | | Heavy chain variable regions | | |
|---|---|---|---|---|---|
| mAb | Mutation sites in light chain CDRs | SEQ ID. | Mutation sites in heavy chain CDRs | SEQ ID. | IC50 (nM) |
| h15H11-1 | E27D, M33L | 55 | D31E, N58L, N100D, T100aD | 32 | 0.47 |
| h15H11-2 | E27D, M33L | 55 | N58L, N100D, T100aD | 33 | 0.48 |
| h15H11-3 | WT | 28 | D31E, N58L, N100D, T100aD | 32 | 0.50 |
| h15H11-4 | WT | 28 | N58L, N100D, T100aD | 33 | 0.51 |
| h15H11-5 | E27D, M33L | 55 | D31E, N58L, N100D | 34 | 0.52 |
| h15H11-6 | E27D, M33L | 55 | N100D, T100aD | 35 | 0.57 |
| h15H11-7 | E27D, M33L | 55 | D31E, N58L, T100aD | 36 | 0.58 |
| h15H11-8 | WT | 28 | N58L, T100aD | 37 | 0.60 |
| h15H11-9 | E27D, M33L | 55 | N58L, T100aD | 37 | 0.61 |
| h15H11-10 | WT | 28 | D31E, N58L, T100aD | 36 | 0.61 |
| h15H11-11 | E27D, M33L | 55 | N58L, N100D | 38 | 0.62 |
| h15H11-12 | WT | 28 | D31E, N58L, N100D | 34 | 0.62 |
| h15H11-13 | E27D, M33L | 55 | D31E, T100aD | 39 | 0.64 |
| h15H11-14 | WT | 28 | N100D, T100aD | 35 | 0.66 |
| h15H11-15 | WT | 28 | N58L, N100D | 38 | 0.66 |
| h15H11-16 | WT | 28 | D31E, T100aD | 39 | 0.68 |
| h15H11-17 | E27D, M33L | 55 | T100aD | 40 | 0.70 |
| h15H11-18 | A25V, E27D, M33L | 56 | T100aD | 40 | 0.74 |
| h15H11-19 | E27D, M33L | 55 | D31E, N100D | 41 | 0.74 |
| h15H11-20 | WT | 28 | T100aD | 40 | 0.77 |

| | | | | | |
|---|---|---|---|---|---|
| 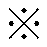 Amino acid positions are according to Kabat numbering. | | | | | |

**[Table 10]**

| | Light chain variable regions | | Heavy chain variable regions | | |
|---|---|---|---|---|---|
| mAb | Mutation sites in light chain CDRs | SEQ ID. | Mutation sites in heavy chain CDRs | SEQ ID. | IC50 (nM) |
| h15H11-21 | E27D, M33L | 55 | D31E, N58L | 42 | 0.80 |
| h15H11-22 | WT | 28 | D31E, N100D | 41 | 0.80 |
| h15H11-23 | E27D, M33L | 55 | D31E, T100aS | 43 | 0.83 |
| h15H11-24 | E27D, M33L | 55 | D31E, N58Q, T100aS | 44 | 0.85 |
| h15H11-25 | WT | 28 | D31E, N58L | 42 | 0.86 |
| h15H11-26 | A25V, E27D, M33L | 56 | N100D | 45 | 0.88 |
| h15H11-27 | A25V, E27D, M33L | 56 | N58L | 46 | 0.91 |
| h15H11-28 | WT | 28 | D31E, T100aS | 43 | 0.91 |
| h15H11-29 | WT | 28 | N58L | 46 | 0.91 |
| h15H11-30 | E27D, M33L | 55 | N100D | 45 | 0.92 |
| h15H11-31 | WT | 28 | D31E, N58Q, T100aS | 44 | 0.93 |
| h15H11-32 | A25V, E27D, M33L | 56 | D31E, T100aS | 43 | 0.95 |
| h15H11-33 | E27D, M33L | 55 | N58Q, T100aS | 47 | 0.98 |
| h15H11-34 | E27D, M33L | 55 | D31E, Y97F, N100Q, T100aS | 48 | 0.99 |
| h15H11-35 | E27D, M33L | 55 | T100aQ | 49 | 1.00 |
| h15H11-36 | E27D, M33L | 55 | S59L | 50 | 1.02 |
| h15H11-37 | A25V, E27D, M33L | 56 | T100aQ | 49 | 1.04 |
| h15H11-38 | A25V, E27D, M33L | 56 | N58K | 51 | 1.04 |
| h15H11-39 | E27D, M33L | 55 | S59Y | 52 | 1.05 |
| h15H11-40 | A25V, E27D, M33L | 56 | S59L | 50 | 1.06 |
| h15H11-41 | E27D, M33L | 55 | Y97F, T100aS | 53 | 1.07 |
| h15H11-42 | WT | 28 | N100K | 54 | 0.83 |
| h15H11-43 | I30F | 57 | WT | 25 | 0.83 |
| h15H11-WT | WT | 28 | WT | 25 | 1.44 |

| | | | | | |
|---|---|---|---|---|---|
| 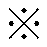 Amino acid positions are according to Kabat numbering. | | | | | |

The sequence numbers of each CDR of each antibody shown in Tables 9 and 10 are shown in Tables 11 and 12.

**[Table 11]**

| mAb | Heavy chain | | | Light chain | | |
|---|---|---|---|---|---|---|
| | CDR1 | CDR2 | CDR3 | CDR1 | CDR2 | CDR3 |
| h15H11-1 | 58 | 59 | 64 | 72 | 9 | 10 |
| h15H11-2 | 4 | 59 | 64 | 72 | 9 | 10 |
| h15H11-3 | 58 | 59 | 64 | 8 | 9 | 10 |
| h15H11-4 | 4 | 59 | 64 | 8 | 9 | 10 |
| h15H11-5 | 58 | 59 | 65 | 72 | 9 | 10 |
| h15H11-6 | 4 | 5 | 64 | 72 | 9 | 10 |
| h15H11-7 | 58 | 59 | 66 | 72 | 9 | 10 |
| h15H11-8 | 4 | 59 | 66 | 8 | 9 | 10 |
| h15H11-9 | 4 | 59 | 66 | 72 | 9 | 10 |
| h15H11-10 | 58 | 59 | 66 | 8 | 9 | 10 |
| h15H11-11 | 4 | 59 | 65 | 72 | 9 | 10 |
| h15H11-12 | 58 | 59 | 65 | 8 | 9 | 10 |
| h15H11-13 | 58 | 5 | 66 | 72 | 9 | 10 |
| h15H11-14 | 4 | 5 | 64 | 8 | 9 | 10 |
| h15H11-15 | 4 | 59 | 65 | 8 | 9 | 10 |
| h15H11-16 | 58 | 5 | 66 | 8 | 9 | 10 |
| h15H11-17 | 4 | 5 | 66 | 72 | 9 | 10 |
| h15H11-18 | 4 | 5 | 66 | 73 | 9 | 10 |
| h15H11-19 | 58 | 5 | 65 | 72 | 9 | 10 |
| h15H11-20 | 4 | 5 | 66 | 8 | 9 | 10 |

**[Table 12]**

| mAb | Heavy chain | | | Light chain | | |
|---|---|---|---|---|---|---|
| | CDR1 | CDR2 | CDR3 | CDR1 | CDR2 | CDR3 |
| h15H11-21 | 58 | 59 | 6 | 72 | 9 | 10 |
| h15H11-22 | 58 | 5 | 65 | 8 | 9 | 10 |
| h15H11-23 | 58 | 5 | 67 | 72 | 9 | 10 |
| h15H11-24 | 58 | 60 | 67 | 72 | 9 | 10 |
| h15H11-25 | 58 | 59 | 6 | 8 | 9 | 10 |
| h15H11-26 | 4 | 5 | 65 | 73 | 9 | 10 |
| h15H11-27 | 4 | 59 | 6 | 73 | 9 | 10 |
| h15H11-28 | 58 | 5 | 67 | 8 | 9 | 10 |
| h15H11-29 | 4 | 59 | 6 | 8 | 9 | 10 |
| h15H11-30 | 4 | 5 | 65 | 72 | 9 | 10 |
| h15H11-31 | 58 | 60 | 67 | 8 | 9 | 10 |
| h15H11-32 | 58 | 5 | 67 | 73 | 9 | 10 |
| h15H11-33 | 4 | 60 | 67 | 72 | 9 | 10 |
| h15H11-34 | 58 | 5 | 68 | 72 | 9 | 10 |
| h15H11-35 | 4 | 5 | 69 | 72 | 9 | 10 |
| h15H11-36 | 4 | 61 | 6 | 72 | 9 | 10 |
| h15H11-37 | 4 | 5 | 69 | 73 | 9 | 10 |
| h15H11-38 | 4 | 62 | 6 | 73 | 9 | 10 |
| h15H11-39 | 4 | 63 | 6 | 72 | 9 | 10 |
| h15H11-40 | 4 | 61 | 6 | 73 | 9 | 10 |
| h15H11-41 | 4 | 5 | 70 | 72 | 9 | 10 |
| h15H11-42 | 4 | 5 | 71 | 8 | 9 | 10 |
| h15H11-43 | 4 | 5 | 6 | 74 | 9 | 10 |
| h15H11-WT | 4 | 5 | 6 | 8 | 9 | 10 |

In addition, the CDR sequences of the antibodies listed in Tables 9 to 12 can be collectively expressed as follows due to their common characteristic.

The CDR1 of the heavy chain is composed of 5 amino acids of Xaa1-Y-N-M-H (wherein Xaa1 is D or E: SEQ ID NO: 75).

The CDR2 of the heavy chain is composed of 17 amino acids of R-I-N-P-K-N-G-V-I-Xaa1-Xaa2-N-E-K-F-K-D (wherein Xaa1 is N, L, Q or K and Xaa2 is S, L or Y: SEQ ID NO: 76).

The CDR3 of the heavy chain is composed of 10 amino acids of S-Y-Xaa1-G-G-Xaa2-Xaa3-D-A-Y (wherein Xaa1 is Y or F, Xaa2 is N, D, Q, or K, and Xaa3 is T, D, S or Q: SEQ ID NO: 77).

The CDR1 of the light chain is composed of 15 amino acids of R-Xaa1-S-Xaa2-S-V-D-N-Y-G-Xaa3-S-F-Xaa4-N (wherein Xaa1 is A or V, Xaa2 is E or D, Xaa3 is I or F, and Xaa4 is M or L: SEQ ID NO: 78).

The CDR2 of the light chain is composed of 7 amino acids of SEQ ID NO: 9.

The CDR3 of the light chain is composed of 9 amino acids of SEQ ID NO: 10.

### Example 8 Production of mutants

Mutants (h15H11-44 to 54) in which various mutations were introduced into the light chain human framework sequence of h15H11-2 (heavy chain variable region: IGHV7-4-1 Q43R, G44S, N58L, N100D, T100aD, R94P (SEQ ID NO: 33)/light chain variable region: IGKV7-3 E27D, M33L, Y36F, V78M (SEQ ID NO: 55)) produced in Example 7 were produced. Affinity evaluation was performed on the produced mutants by the method described in Example 5. The results obtained are shown in Table 13.

A mutant having the heavy chain variable region represented by IGHV7-4-1 Q43R, G44S, N58L, N100D, T100aD, R94P (SEQ ID NO: 33) and the light chain variable region represented by IGKV4-1 (SEQ ID NO: 18) produced in Example 3 was produced. New mutants (h15H11-55 to 67) were produced in which various mutations were introduced into the light chain human framework sequence of the produced mutants, and affinity evaluation was performed on the produced mutants by the method described in Example 5. The results obtained are shown in Table 14.

**[Table 13]**

| | Heavy chain variable regions | | | Light chain variable regions | | | |
|---|---|---|---|---|---|---|---|
| mAb | Framework | Heavy chain mutations | SEQ ID. | Framework | Light chain mutations | SEQ ID. | IC50 (nM) |
| h15H11-44 | IGHV7-4-1 | Q43R, G44S, N58L, N100D, T100aD, R94P | 33 | IGKV 7-3 | E27D, M33L, Y36F, V78M, N81E | 79 | 0.51 |
| h15H11-45 | | | | | E27D, M33L, Y36F, V78M, N81A | 80 | 0.50 |
| h15H11-46 | | | | | E27D, M33L, Y36F, V78M, N81Q | 81 | 0.50 |
| h15H11-47 | | | | | E27D, M33L, Y36F, V78M, N81F | 82 | 0.53 |
| h15H11-48 | | | | | E27D, M33L, Y36F, V78M, N81L | 83 | 0.51 |
| h15H11-49 | | | | | E27D, M33L, Y36F, V78M, N81V | 84 | 0.62 |
| h15H11-50 | | | | | E27D, M33L, Y36F, V78M, N81I | 85 | 0.55 |
| h15H11-51 | | | | | E27D, M33L, Y36F, V78M, N81D | 86 | 0.63 |
| h15H11-52 | | | | | E27D, M33L, Y36F, V78M, N81R | 87 | 0.50 |
| h15H11-53 | | | | | E27D, M33L, Y36F, V78M, N81G | 88 | 0.55 |
| h15H11-54 | | | | | E27D, M33L, Y36F, V78M, D82P | 89 | 0.72 |

| | | | | | | | |
|---|---|---|---|---|---|---|---|
| 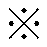 Amino acid positions are according to Kabat numbering. | | | | | | | |

**[Table 14]**

| | Heavy chain variable regions | | | Light chain variable regions | | | |
|---|---|---|---|---|---|---|---|
| mAb | Framework | Heavy chain mutations | SEQ ID. | Framework | Light chain mutations | SEQ ID. | IC50 (nM) |
| h15H11-55 | IGHV7-4-1 | Q43R, G44S, N58L, N100D, T100aD, R94P | 33 | IGKV4-1 | Y36F, L78M, V85M | 90 | 0.82 |
| h15H11-56 | | | | | Y36F, S76N, S77P, L78M | 91 | 0.85 |
| h15H11-57 | | | | | Y36F, L78M | 92 | 0.89 |
| h15H11-58 | | | | | E27D, M33L, Y36F, L78M, V85M | 93 | 0.78 |
| h15H11-59 | | | | | E27D, M33L, Y36F, S76N, S77P, L78M | 94 | 0.80 |
| h15H11-60 | | | | | E27D, M33L, Y36F, L78M | 95 | 0.79 |
| h15H11-61 | | | | | E27D, M33L, Y36F, S76N, S77P, L78M, Q79E, V83T, V85N | 96 | 0.79 |
| h15H11-62 | | | | | E27D, M33L, Y36F, L78M, Q79E | 97 | 0.73 |
| h15H11-63 | | | | | E27D, M33L, Y36F, L78M, V83T | 98 | 0.64 |
| h15H11-64 | | | | | E27D, M33L, Y36F, D60A, L78M | 99 | 0.68 |
| h15H11-65 | | | | | E27D, M33L, Y36F, D60A, L78M, V85M | 100 | 0.75 |
| h15H11-66 | | | | | M4L, D9A, E27D, M33L, Y36F, D60A, L78M | 101 | 0.53 |
| h15H11-67 | | | | | M4L, D9A, E17Q, E27D, M33L, Y36F, D60A, L78M | 102 | 0.46 |

| | | | | | | | |
|---|---|---|---|---|---|---|---|
| 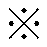 Amino acid positions are according to Kabat numbering. | | | | | | | |

The sequence numbers of each CDR of each antibody shown in Tables 13 and 14 are shown. h15H11-44 to h15H11-54, h15H11-58 to h15H11-67 have the heavy chain CDR1 sequence of SEQ ID NO: 4, the heavy chain CDR2 sequence of SEQ ID NO: 59 and the heavy chain CDR3 sequence of SEQ ID NO: 64 and the light chain CDR1 sequence of SEQ ID NO: 72, the light chain CDR2 sequence of SEQ ID NO: 9 and the light chain CDR3 sequence of SEQ ID NO: 10. h15H11-55 to h15H11-57 have the heavy chain CDR1 sequence of SEQ ID NO: 4, the heavy chain CDR2 sequence of SEQ ID NO: 59 and the heavy chain CDR3 sequence of SEQ ID NO: 64 and the light chain CDR1 sequence of SEQ ID NO: 8, the light chain CDR2 sequence of SEQ ID NO: 9 and the light chain CDR3 sequence of SEQ ID NO: 10. Similar contents are also shown in Table 15.

**[Table 15]**

| mAb | Heavy chain | | | Light chain | | |
|---|---|---|---|---|---|---|
| | CDR1 | CDR2 | CDR3 | CDR1 | CDR2 | CDR3 |
| h15H11-44 to h15H11-54 and h15H11-58 to h15H11-67 | 4 | 59 | 64 | 72 | 9 | 10 |
| h15H11-55 to h15H11-57 | 4 | 59 | 64 | 8 | 9 | 10 |

In addition, the heavy chain variable region sequences of the antibodies shown in Tables 13 and 14 are represented by SEQ ID NO: 33, and each light chain variable region sequence can be collectively expressed as follows due to their common characteristic.

The light chain variable region sequences of h15H11-44 to h15H11-53 include the sequences in which the amino acid residue at position N81 according to Kabat numbering of SEQ ID NO: 55 (IGKV7-3 (E27D, M33L, Y36F, V78M)) is replaced with E, A, Q, F, L, V, I, D, R or G.

The light chain variable region sequences of h15H11-58 to h15H11-65 are expressed as the sequences in which 1 to 5 amino acid sequences of positions 76, 77, 79, 83, and 85 according to Kabat numbering of SEQ ID NO: 95 (IGKV4-1 (E27D, M33L, Y36F, L78M)) may be mutated.

### Example 9: Evaluation of drug efficacy

### (9-1) Preparation of rat partial sciatic nerve ligation models

Under isoflurane anesthesia, the skin of the upper left thigh of the rat was incised and the muscles were cut to expose the sciatic nerve. Approximately half of the sciatic nerve was tightly ligated with nylon thread and the muscles and skin were sutured. This was the surgery side. The right leg was treated in the same manner, except for scinatic nerve ligation, and this was the sham surgery side.

### (9-2) Evaluation of drug efficacy of intravenous administration

Approximately 2 weeks after surgery, the effect on mechanical allodynia was evaluated in the rats prepared as described in (9-1) by von Frey filaments. The rats were placed in plastic cages on wire mesh, and evaluations were performed after habituation. Before and after administration of the antibody, a von Frey filament (0.4 to 26 g) was pressed to the plantar surface of the rat in order starting from the thin filament. The pressure value of the smallest filament at which the rat exhibits withdrawal behavior was defined as the pain threshold. Pain thresholds were evaluated for the left and right hindlimbs. The animals with pain thresholds of 0.6 to 2 g after surgery and pain thresholds of 8 to 15 g on the sham surgery side were selected to drug efficacy evaluation. The antibodies were prepared at 5, 15, and 50 mg/kg in physiological saline and were administered intravenously. Five hours after administration, the pain thresholds of the left and right hindlimbs were evaluated. The % reversal value was calculated from the following formula and compared as the pain suppressing effect of the compounds. % reversal value = (logarithm of pain threshold after administration on surgery side - logarithm of pain threshold before administration on surgery side)/(logarithm of pain threshold before administration on sham surgery side - logarithm of pain threshold before administration on surgery side)

The results for h12H4 described in Example 4 of Patent Literature: 8 and h15H11-66 included in the present invention are shown in FIG. 3. h15H11-66 showed significant drug efficacy against pain when administered intravenously at a dose of 5 mg/kg. Intravenous administration of h15H11-66 at a dose of 5 mg/kg showed similar analgesic effects as intravenous administration of h12H4 at a dose of 50 mg/kg.

### [INDUSTRIAL APPLICABILITY]

A monoclonal antibody or its antibody fragment of the present invention may be used to detect Navi. 7 in biological samples. In addition, a pharmaceutical composition comprising a monoclonal antibody or its antibody fragment of the present invention, is great useful as a medicine for treating or preventing a Navi. 7 related disease.

## Claims

1. A monoclonal antibody or its antibody fragment that binds to Nav1.7, having;
a heavy chain variable region including
a CDR1 having the amino acid sequence of SEQ ID NO: 75,
a CDR2 having the amino acid sequence of SEQ ID NO: 76 and
a CDR3 having the amino acid sequence of SEQ ID NO: 77 and
a light chain variable region including
a CDR1 having the amino acid sequence of SEQ ID NO: 78,
a CDR2 having the amino acid sequence of SEQ ID NO: 9 and
a CDR3 having the amino acid sequence of SEQ ID NO: 10.

2. The monoclonal antibody or its antibody fragment according to claim 1, having;
a heavy chain variable region including
a CDR1 having the amino acid sequence of SEQ ID NO: 4 or 58,
a CDR2 having the amino acid sequence of any one of SEQ ID NOs: 5, 59 to 63 and
a CDR3 having the amino acid sequence of any one of SEQ ID NOs: 6, 64 to 71, and
a light chain variable region including
a CDR1 having the amino acid sequence of any one of SEQ IDs NO: 8, 72 to 74,
a CDR2 having the amino acid sequence of SEQ ID NO: 9 and
a CDR3 having the amino acid sequence of SEQ ID NO: 10.

3. The monoclonal antibody or its antibody fragment according to claim 2, having
1) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 64 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
2) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 64 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
3) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 64 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
4) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 64 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
5) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 65 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
6) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 64 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
7) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 66 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
8) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 66 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
9) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 66 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
10) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 66 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
11) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 65 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
12) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 65 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
13) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 66 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
14) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NOs: 64 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
15) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 65 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
16) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 66 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
17) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 66 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
18) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 66 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
19) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 65 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
20) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NOs: 66 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
21) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 6 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
22) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 65 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
23) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 67 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
24) a heavy chain variable region including
a CDR1 having the amino acid sequence of SEQ ID NO: 58,
a CDR2 having the amino acid sequence of SEQ ID NO: 60 and
a CDR3 having the amino acid sequence of SEQ ID NO: 67 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
25) a heavy chain variable region including
a CDR1 having the amino acid sequence of SEQ ID NO: 58,
a CDR2 having the amino acid sequence of SEQ ID NO: 59 and
a CDR3 having the amino acid sequence of SEQ ID NO: 6 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
26) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 65 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
27) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 6 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
28) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 67 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
29) a heavy chain variable region including
a CDR1 having the amino acid sequence of SEQ ID NO: 4,
a CDR2 having the amino acid sequence of SEQ ID NO: 59 and
a CDR3 having the amino acid sequence of SEQ ID NO: 6 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
30) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 65 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
31) a heavy chain variable region including
a CDR1 having the amino acid sequence of SEQ ID NO: 58,
a CDR2 having the amino acid sequence of SEQ ID NO: 60 and
a CDR3 having the amino acid sequence of SEQ ID NO: 67 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
32) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 67 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
33) a heavy chain variable region including
a CDR1 having the amino acid sequence of SEQ ID NO: 4,
a CDR2 having the amino acid sequence of SEQ ID NO: 60 and
a CDR3 having the amino acid sequence of SEQ ID NO: 67 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
34) a heavy chain variable region including
a CDR1 having the amino acid sequence of SEQ ID NO: 58,
a CDR2 having the amino acid sequence of SEQ ID NO: 5 and
a CDR3 having the amino acid sequence of SEQ ID NO: 68 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
35) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 69 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
36) a heavy chain variable region including
a CDR1 having the amino acid sequence of SEQ ID NO: 4,
a CDR2 having the amino acid sequence of SEQ ID NO: 61 and
a CDR3 having the amino acid sequence of SEQ ID NO: 6 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
37) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 69 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
38) a heavy chain variable region including
a CDR1 having the amino acid sequence of SEQ ID NO: 4,
a CDR2 having the amino acid sequence of SEQ ID NO: 62 and
a CDR3 having the amino acid sequence of SEQ ID NO: 6 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
39) a heavy chain variable region including
a CDR1 having the amino acid sequence of SEQ ID NO: 4,
a CDR2 having the amino acid sequence of SEQ ID NO: 63 and
a CDR3 having the amino acid sequence of SEQ ID NO: 6 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
40) a heavy chain variable region including
a CDR1 having the amino acid sequence of SEQ ID NO: 4,
a CDR2 having the amino acid sequence of SEQ ID NO: 61 and
a CDR3 having the amino acid sequence of SEQ ID NO: 6 and
a light chain variable region including
a CDR1 having the amino acid sequence of SEQ ID NO: 73,
a CDR2 having the amino acid sequence of SEQ ID NO: 9 and
a CDR3 having the amino acid sequence of SEQ ID NO: 10;
41) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 70 and
a light chain variable region including
a CDR1 having the amino acid sequence of SEQ ID NO: 72,
a CDR2 having the amino acid sequence of SEQ ID NO: 9 and
a CDR3 having the amino acid sequence of SEQ ID NO: 10;
42) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 71 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10;
43) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 6 and
a light chain variable region including
a CDR1 having the amino acid sequence of SEQ ID NO: 74,
a CDR2 having the amino acid sequence of SEQ ID NO: 9 and
a CDR3 having the amino acid sequence of SEQ ID NO: 10; or
44) a heavy chain variable region including
a CDR1 having the amino acid sequence of SEQ ID NO: 4,
a CDR2 having the amino acid sequence of SEQ ID NO: 5 and
a CDR3 having the amino acid sequence of SEQ ID NO: 6 and
a light chain variable region including
a CDR1 having the amino acid sequence of SEQ ID NO: 8,
a CDR2 having the amino acid sequence of SEQ ID NO: 9 and
a CDR3 having the amino acid sequence of SEQ ID NO: 10.

4. The monoclonal antibody or its antibody fragment according to any one of claims 1 to 3 wherein the monoclonal antibody is the humanized monoclonal antibody.

5. The monoclonal antibody or its antibody fragment according to any one of claims 1 to 4, having
a heavy chain variable region having
the amino acid sequence of any one of SEQ ID NOs: 25, 32 to 54 or
the amino acid sequence at least 95% identical to the amino acid sequence of any one of SEQ ID NOs: 25, 32 to 54, and
a light chain variable region having
the amino acid sequence of any one of SEQ ID NOs: 28, 55 to 57, 79 to 102 or
the amino acid sequence at least 95% identical to the amino acid sequence of any one of SEQ ID NOs: 28, 55 to 57, 79 to 102.

6. The monoclonal antibody or its antibody fragment according to claim 5, having
1) a heavy chain variable region having the amino acid sequence of SEQ ID NO: 32, and a light chain variable region having the amino acid sequence of SEQ ID NO: 55;
2) a heavy chain variable region having the amino acid sequence of SEQ ID NO: 33, and a light chain variable region having the amino acid sequence of SEQ ID NO: 55;
3) a heavy chain variable region having the amino acid sequence of SEQ ID NO: 32, and a light chain variable region having the amino acid sequence of SEQ ID NO: 28;
4) a heavy chain variable region having the amino acid sequence of SEQ ID NO: 33, and a light chain variable region having the amino acid sequence of SEQ ID NO: 28;
5) a heavy chain variable region having the amino acid sequence of SEQ ID NO: 34, and a light chain variable region having the amino acid sequence of SEQ ID NO: 55;
6) a heavy chain variable region having the amino acid sequence of SEQ ID NO: 35, and a light chain variable region having the amino acid sequence of SEQ ID NO: 55;
7) a heavy chain variable region having the amino acid sequence of SEQ ID NO: 36, and a light chain variable region having the amino acid sequence of SEQ ID NO: 55;
8) a heavy chain variable region having the amino acid sequence of SEQ ID NO: 37, and a light chain variable region having the amino acid sequence of SEQ ID NO: 28;
9) a heavy chain variable region having the amino acid sequence of SEQ ID NO: 37, and a light chain variable region having the amino acid sequence of SEQ ID NO: 55;
10) a heavy chain variable region having the amino acid sequence of SEQ ID NO: 36, and a light chain variable region having the amino acid sequence of SEQ ID NO: 28;
11) a heavy chain variable region having the amino acid sequence of SEQ ID NO: 38, and a light chain variable region having the amino acid sequence of SEQ ID NO: 55;
12) a heavy chain variable region having the amino acid sequence of SEQ ID NO: 34, and a light chain variable region having the amino acid sequence of SEQ ID NO: 28;
13) a heavy chain variable region having the amino acid sequence of SEQ ID NO: 39, and a light chain variable region having the amino acid sequence of SEQ ID NO: 55;
14) a heavy chain variable region having the amino acid sequence of SEQ ID NO: 35, and a light chain variable region having the amino acid sequence of SEQ ID NO: 28;
15) a heavy chain variable region having the amino acid sequence of SEQ ID NO: 38, and a light chain variable region having the amino acid sequence of SEQ ID NO: 28;
16) a heavy chain variable region having the amino acid sequence of SEQ ID NO: 39, and a light chain variable region having the amino acid sequence of SEQ ID NO: 28;
17) a heavy chain variable region having the amino acid sequence of SEQ ID NO: 40, and a light chain variable region having the amino acid sequence of SEQ ID NO: 55;
18) a heavy chain variable region having the amino acid sequence of SEQ ID NO: 40, and a light chain variable region having the amino acid sequence of SEQ ID NO: 56;
19) a heavy chain variable region having the amino acid sequence of SEQ ID NO: 41, and a light chain variable region having the amino acid sequence of SEQ ID NO: 55;
20) a heavy chain variable region having the amino acid sequence of SEQ ID NO: 40, and a light chain variable region having the amino acid sequence of SEQ ID NO: 28;
21) a heavy chain variable region having the amino acid sequence of SEQ ID NO: 42, and a light chain variable region having the amino acid sequence of SEQ ID NO: 55;
22) a heavy chain variable region having the amino acid sequence of SEQ ID NO: 41, and a light chain variable region having the amino acid sequence of SEQ ID NO: 28;
23) a heavy chain variable region having the amino acid sequence of SEQ ID NO: 43, and a light chain variable region having the amino acid sequence of SEQ ID NO: 55;
24) a heavy chain variable region having the amino acid sequence of SEQ ID NO: 44, and a light chain variable region having the amino acid sequence of SEQ ID NO: 55;
25) a heavy chain variable region having the amino acid sequence of SEQ ID NO: 42, and a light chain variable region having the amino acid sequence of SEQ ID NO: 28;
26) a heavy chain variable region having the amino acid sequence of SEQ ID NO: 45, and a light chain variable region having the amino acid sequence of SEQ ID NO: 56;
27) a heavy chain variable region having the amino acid sequence of SEQ ID NO: 46, and a light chain variable region having the amino acid sequence of SEQ ID NO: 56;
28) a heavy chain variable region having the amino acid sequence of SEQ ID NO: 43, and a light chain variable region having the amino acid sequence of SEQ ID NO: 28;
29) a heavy chain variable region having the amino acid sequence of SEQ ID NO: 46, and a light chain variable region having the amino acid sequence of SEQ ID NO: 28;
30) a heavy chain variable region having the amino acid sequence of SEQ ID NO: 45, and a light chain variable region having the amino acid sequence of SEQ ID NO: 55;
31) a heavy chain variable region having the amino acid sequence of SEQ ID NO: 44, and a light chain variable region having the amino acid sequence of SEQ ID NO: 28;
32) a heavy chain variable region having the amino acid sequence of SEQ ID NO: 43, and a light chain variable region having the amino acid sequence of SEQ ID NO: 56;
33) a heavy chain variable region having the amino acid sequence of SEQ ID NO: 47, and a light chain variable region having the amino acid sequence of SEQ ID NO: 55;
34) a heavy chain variable region having the amino acid sequence of SEQ ID NO: 48, and a light chain variable region having the amino acid sequence of SEQ ID NO: 55;
35) a heavy chain variable region having the amino acid sequence of SEQ ID NO: 49, and a light chain variable region having the amino acid sequence of SEQ ID NO: 55;
36) a heavy chain variable region having the amino acid sequence of SEQ ID NO: 50, and a light chain variable region having the amino acid sequence of SEQ ID NO: 55;
37) a heavy chain variable region having the amino acid sequence of SEQ ID NO: 49, and a light chain variable region having the amino acid sequence of SEQ ID NO: 56;
38) a heavy chain variable region having the amino acid sequence of SEQ ID NO: 51, and a light chain variable region having the amino acid sequence of SEQ ID NO: 56;
39) a heavy chain variable region having the amino acid sequence of SEQ ID NO: 52, and a light chain variable region having the amino acid sequence of SEQ ID NO: 55;
40) a heavy chain variable region having the amino acid sequence of SEQ ID NO: 50, and a light chain variable region having the amino acid sequence of SEQ ID NO: 56;
41) a heavy chain variable region having the amino acid sequence of SEQ ID NO: 53, and a light chain variable region having the amino acid sequence of SEQ ID NO: 55;
42) a heavy chain variable region having the amino acid sequence of SEQ ID NO: 54, and a light chain variable region having the amino acid sequence of SEQ ID NO: 28;
43) a heavy chain variable region having the amino acid sequence of SEQ ID NO: 25, and a light chain variable region having the amino acid sequence of SEQ ID NO: 57;
44) a heavy chain variable region having the amino acid sequence of SEQ ID NO: 25, and a light chain variable region having the amino acid sequence of SEQ ID NO: 28;
45) a heavy chain variable region having the amino acid sequence of SEQ ID NO: 33, and a light chain variable region having the amino acid sequence of SEQ ID NO: 79;
46) a heavy chain variable region having the amino acid sequence of SEQ ID NO: 33, and a light chain variable region having the amino acid sequence of SEQ ID NO: 80;
47) a heavy chain variable region having the amino acid sequence of SEQ ID NO: 33, and a light chain variable region having the amino acid sequence of SEQ ID NO: 88;
48) a heavy chain variable region having the amino acid sequence of SEQ ID NO: 33, and a light chain variable region having the amino acid sequence of SEQ ID NO: 101;
49) a heavy chain variable region having the amino acid sequence of SEQ ID NO: 33, and a light chain variable region having the amino acid sequence of SEQ ID NO: 102;
50) a heavy chain variable region having the amino acid sequence of SEQ ID NO: 33, and a light chain variable region having the amino acid sequence of SEQ ID NO: 81;
51) a heavy chain variable region having the amino acid sequence of SEQ ID NO: 33, and a light chain variable region having the amino acid sequence of SEQ ID NO: 82;
52) a heavy chain variable region having the amino acid sequence of SEQ ID NO: 33, and a light chain variable region having the amino acid sequence of SEQ ID NO: 83;
53) a heavy chain variable region having the amino acid sequence of SEQ ID NO: 33, and a light chain variable region having the amino acid sequence of SEQ ID NO: 84;
54) a heavy chain variable region having the amino acid sequence of SEQ ID NO: 33, and a light chain variable region having the amino acid sequence of SEQ ID NO: 85;
55) a heavy chain variable region having the amino acid sequence of SEQ ID NO: 33, and a light chain variable region having the amino acid sequence of SEQ ID NO: 86;
56) a heavy chain variable region having the amino acid sequence of SEQ ID NO: 33, and a light chain variable region having the amino acid sequence of SEQ ID NO: 87;
57) a heavy chain variable region having the amino acid sequence of SEQ ID NO: 33, and a light chain variable region having the amino acid sequence of SEQ ID NO: 89;
58) a heavy chain variable region having the amino acid sequence of SEQ ID NO: 33, and a light chain variable region having the amino acid sequence of SEQ ID NO: 90;
59) a heavy chain variable region having the amino acid sequence of SEQ ID NO: 33, and a light chain variable region having the amino acid sequence of SEQ ID NO: 91;
60) a heavy chain variable region having the amino acid sequence of SEQ ID NO: 33, and a light chain variable region having the amino acid sequence of SEQ ID NO: 92;
61) a heavy chain variable region having the amino acid sequence of SEQ ID NO: 33, and a light chain variable region having the amino acid sequence of SEQ ID NO: 93;
62) a heavy chain variable region having the amino acid sequence of SEQ ID NO: 33, and a light chain variable region having the amino acid sequence of SEQ ID NO: 94;
63) a heavy chain variable region having the amino acid sequence of SEQ ID NO: 33, and a light chain variable region having the amino acid sequence of SEQ ID NO: 95;
64) a heavy chain variable region having the amino acid sequence of SEQ ID NO: 33, and a light chain variable region having the amino acid sequence of SEQ ID NO: 96;
65) a heavy chain variable region having the amino acid sequence of SEQ ID NO: 33, and a light chain variable region having the amino acid sequence of SEQ ID NO: 97;
66) a heavy chain variable region having the amino acid sequence of SEQ ID NO: 33, and a light chain variable region having the amino acid sequence of SEQ ID NO: 98;
67) a heavy chain variable region having the amino acid sequence of SEQ ID NO: 33, and a light chain variable region having the amino acid sequence of SEQ ID NO: 99; or
68) a heavy chain variable region having the amino acid sequence of SEQ ID NO: 33, and a light chain variable region having the amino acid sequence of SEQ ID NO: 100.

7. The monoclonal antibody or its antibody fragment according to any one of claims 1 to 6, further having
a heavy chain constant region having the amino acid sequence of SEQ ID NO: 11 and
a light chain constant region having the amino acid sequence of SEQ ID NO: 12.

8. A pharmaceutical composition comprising the monoclonal antibody or its antibody fragment according to any one of claims 1 to 7.

9. The pharmaceutical composition according to claim 8, which is a therapeutic and/or preventive agent for pain and/or pruritus.

10. A polynucleotide encoding a heavy chain variable region of the antibody according to claim 5 or 6 and optionally encoding a heavy chain constant region of the antibody according to claim 7.

11. A polynucleotide encoding a light chain variable region of the antibody according to claim 5 or 6 and optionally encoding a light chain constant region of the antibody according to claim 7.

12. An expression vector including the polynucleotide according to claim 10 and/or 11.
